# NEUE EUROPÄISCHE PATENTSCHRIFT

(11) **EP 0 769 954 B2**
(45) Veröffentlichungstag und Bekanntmachung des Hinweises auf die Entscheidung über den Einspruch: **09.03.2011**
(45) Hinweis auf die Patenterteilung: 30.07.2003
(21) Anmeldenummer: 96910855.4
(22) Anmeldetag: 06.05.1996
(51) Int. Cl.: A61K 35/14, C12Q 1/68, C12Q 1/70, G01N 33/96, A61L 2/00, G01N 33/569, G01N 33/576

(54) **QUALITÄTSGESICHERTES ARZNEIMITTEL, ENTHALTEND EIN ODER MEHRERE PLASMADERIVATE**
MEDICAMENT OF ASSURED QUALITY, CONTAINING ONE OR MORE PLASMA DERIVATIVES
MEDICAMENT DE QUALITE GARANTIE CONTENANT UN OU PLUSIEURS DERIVES PLASMATIQUES

(30) Priorität: 08.05.1995 AT 77895
(43) Veröffentlichungstag der Anmeldung: 02.05.1997
(73) Patentinhaber: Baxter Aktiengesellschaft, 1221 Wien (AT)
(72) Erfinder: EIBL, Johann, A-1180 Wien (AT); SCHWARZ, Otto, A-1190 Wien (AT); DORNER, Friedrich, A-1230 Wien (AT); IGEL, Herwig, A-1190 Wien (AT)
(74) Vertreter: Pawloy, Peter Michael
(86) Internationale Anmeldenummer: PCT/AT1996/000089
(87) Internationale Veröffentlichungsnummer: WO 1996/035437

(56) Entgegenhaltungen:
- EP-A- 0 447 984
- EP-A- 0 519 901
- EP-A- 0 590 327
- EP-A- 0 714 988
- WO-A-92/01714
- WO-A-93/23573
- JOURNAL OF MEDICAL VIROLOGY, Bd. 43, Nr. 1, 1994, NEW YORK, N.Y., US, Seiten 72-76, XP000577184 J. SALDANHA ET AL.: "A SENSITIVE PCR METHOD FOR DETECTING HCV RNA IN PLASMA POOLS, BLOOD PRODUCTS, AND SINGLE DONATIONS."
- JOURNAL OF CLINICAL MICROBIOLOGY, Bd. 31, Nr. 2, Februar 1993, WASHINGTON, DC, US, Seiten 323-328, XP000577180 F. MCOMISH ET AL.: "DETECTION OF PARVOVIRUS B19 IN DONATED BLOOD: A MODEL SYSTEM FOR SCREENING BY POLYMERASE CHAIN REACTION."
- THE LANCET, Bd. 335, Nr. 8703, 16.Juni 1990, LONDON GB, Seite 1473 XP002009939 J.A. GARSON ET AL.: "DETECTION BY PCR OF HEPATITIS C VIRUS IN FACTOR VIII CONCENTRATES."
- DORNER ET AL DEV. BIOL. STAND. Bd. 81, 1993, Seiten 137 - 143
- Vox Sang. 67 (1994) S2, Abstracts No. 70, 71, 213 and 214

## Beschreibung

Die Erfindung betrifft verfahren zur Herstellung eines Arzneimittels, enthaltend ein oder mehrere Plasmaderivate als Wirksubstanz oder Hilfsstoff, qualitätsgesichertes Ausgangsmaterial zur Herstellung solcher Arzneimittel. Die Erfindung betrifft ebenso ein Verfahren zur Herstellung von qualitätsgesicherten Ausgangsmaterialien, insbesondere qualitätsgesicherten Plasmapools.

Menschliches Plasma ist als pharmazeutisches Präparat bzw. als Ausgangsmaterial zur Herstellung von Plasmaderivaten von außerordentlicher klinischer Bedeutung, insbesondere zur Substitutionstherapie bei angeborenem oder erworbenen Mangel an Plasmakomponenten. Bei der Verwendung von menschlichen Plasma ist aber darauf zu achten, daß keine infektiösen Agentien enthalten sind, die mit dem pharmazeutischen Präparat bzw. mit den Plasmaderivaten übertragen werden können. Zu den möglicherweise im Blut vorkommenden infektiösen Agentien zählen vor allem Viren, die durch Blut übertragbar sind (haematogene Viren), z.B. HI-Viren oder Hepatitisviren (Hepatitis A, B, C oder non-A-non-B), aber auch Parvovirus.

Aufgrund des großen Bedarfs an Arzneimitteln enthaltend Plasmaderivate ist die ökonomische Herstellung dieser Arzneimittel nur im industriellen Maßstab möglich.

Plasma wird von Spendern erhalten und zur Herstellung von pharmazeutischen Präparaten gepoolt. Die Größe eines üblichen Pools beträgt etwa 2000-6000 Einzelplasmaspenden. Dabei besteht das Risiko, daß durch eine einzelne Virus-kontaminierte Plasmaspende der gesamte Plasmapool kontaminiert wird.

Obwohl es bereits gegen Ende der ersten Hälfte des 20. Jahrhunderts gelungen war, menschliches Albumin durch Erhitzen zu einem virussicheren Präparat zu machen, war dies bei allen anderen aus Plasma gewinnbaren Arzneimitteln wegen ihrer Empfindlichkeit gegenüber Hitze zunächst nicht möglich. Bis heute sind bei millionenfacher Anwendung von adäquat hergestelltem Albumin nie Infektionen mit beim Menschen im Blut auftretenden Viren vorgekommen.

Im Gegensatz hierzu wurde bei vielen anderen aus Plasma hergestellten Arzneimitteln immer wieder über Virusinfektionen, insbesondere mit Hepatitisviren, berichtet und seit den 80-iger Jahren im großen Umfang auch über Infektionen mit Aids-Virus.

Um 1980 wurden erstmals bei entsprechend stabilisierten Faktor VIII-Konzentraten Hitzebehandlungen durchgeführt, mit der Absicht, hierdurch Inaktivierungen von Viruskontaminanten zu erreichen. Zunächst mußte aber ein großer Verlust an Faktor VII-Aktivität in Kauf genommen werden bzw. blieb das tatsächliche Inaktivierungspotential zunächst unbekannt.

Durch Verbesserung der Hitzeinaktivierungsverfahren und anderen neuen Inaktivierungsverfahren konnten schließlich Arzneimittel aus Plasma hergestellt werden, die in den meisten Fällen zu keinen Virusinfektionen beim Empfänger führten. Hand in Hand mit dieser Entwicklung ging auch die Verbesserung der Spender- und Spendenauswahl mit der Zielrichtung, jene Spender und Spenden auszuschließen, bei denen der Verdacht einer Virämie und damit eines virushältigen Plasmas bestand.

Seit längerer Zeit wird entweder durch Antigennachweis oder durch Antikörpernachweis von bzw. gegen ein bestimmtes Virus im Blut versucht, solche Spenden, die ein positives Ergebnis liefern, auszuschließen und sie nicht in einen größeren Plasmapool einzubringen, der als Ausgangsmaterial für die Herstellung von Blutprodukten dienen soll. Bei Einzelspendern, die in allen Untersuchungen keine Krankheitssymptome oder pathologische Untersuchungsergebnisse aufweisen, trotzdem aber bestimmte Viren in ihrem Blut sogar in hoher Konzentration über längere Zeiträume beherbergen können, kann nunmehr das Auftreten solcher Virämien durch ein bestimmtes Virus mit Hilfe einer Amplifikationsmethode eindeutig nachgewiesen werden.

Durch das Poolen von Plasmaeinheiten wird zwar naturgemäß eine einzelne mit Viren kontaminierte Plasmaspende verdünnt, der Nachweis von viralen Genomsequenzen mit Hilfe von Amplifikationsreaktionen ist aber so empfindlich, daß selbst in den Verdünnungen Virusgenome bzw. deren Sequenzen eindeutig bestimmbar sind und falls sie, wie oben erwähnt, unter eine bestimmte Bestimmbarkeitsgrenze fallen, dann nicht mehr eine klinische Relevanz im Sinne der Möglichkeit einer Infektion aufweisen.

Die EG-Richtlinie gemäß dem "EEC Regulatory Document Note for Guidance", Guidelines for Medicinal Products Derived from Human Blood and Plasma (Biologicals 1992, 20: 159-164) schlägt ein Qualitätssicherheitssystem zur Kontrolle der Plasmaspender bzw. Plasmaspenden vor. Demnach muß jede Plasmaspende mit validierten Tests auf die Abwesenheit von Virus-Markern, wie Hepatitis-B Antigen, HIV-1- und HIV-2-Antikörpern untersucht werden, da diese indikativ für eine entsprechende virale Infektion des Plasmaspenders sind. Tests zum Ausschluß Bestimmung einer Hepatitis C-Infektion sollen gleichfalls vorgenommen werden.

Gemäß der europäischen Pharmacopoeia ist beschrieben, daß spezielle Tests zur Bestimmung von Hepatitis B-Oberflächenantigen, für Hepatitis C-Virus-Antikörper und für HIV-Antikörper an jeder Spende durchgeführt werden sollen. (European Pharmacopoeia, 2. Ausgabe, Teil II, 1994, Seiten 853 bis 853-4).

Eine FDA-Richtlinie vom 14.3.1995 sieht die PCR-Testung an einem Endprodukt (Immunglobulin-Produkt) als zusätzlichen Sicherheitsfaktor vor.

Trotz der vorgeschlagenen Tests wird in der EG-Richtlinie betont, daß die Sicherheit von einzelnen Plasmaspenden nur durch eine Kontrolle dieser Virus-Marker nicht ausreichend ist. Auch wenn die Abwesenheit der genannten Marker in einer Plasmaprobe bestätigt wird, ist eine Virämie des Spenders nicht auszuschließen. Virale Antigene und entsprechende Antikörper sind nämlich nicht sofort nach der Infektion nachweisbar, die ersten Marker für eine virale Infektion treten oft erst nach Wochen oder Monaten nach dem Kontakt mit infektiösem Material auf. Dieser kritische Zeitraum nach Infektion und vor dem Auftreten von Antikörpern wird allgemein als "Window-Periode" bezeichnet. Der Zeitpunkt nach Infektion, bei dem die ersten viralen Marker nachweisbar sind, ist jedoch von Virus zu Virus verschieden.

Darüberhinaus wurde auch bekannt, daß für manche aus Plasma hergestellte Arzneimittel es im Rahmen des Herstellungsverfahrens zu einer Abreicherung bzw. Inaktivierung von Viren kommt und solche Produkte von sich aus in hohem Ausmaß virussicher sind.

Obwohl Virusinaktivierungen von Plasmaderivaten im industriellen Ausmaß äußerst erfolgreich durchgeführt wurden, kam es in seltenen Fällen trotzdem zu Übertragungen haematogener Viren wie AIDS, Hepatitis A, B, C-Virus, wodurch angenommen werden muß, daß Hersteller trotz Anwendung einer gleichbleibenden Herstellungsmethode bei einigen wenigen Herstellungschargen viruskontaminierte Produkte erzeugten (Lancet 340, 305-306 (1992); MMWR 43 (28), 505-509 (1994); Thromb.Haemost. 68, 781 (1992). Die Ursache dafür ist wahrscheinlich in einer von übermäßigen Kontamination bestimmter Ausgangschargen zu suchen. Da bei der Gewinnung des Plasmas nur indirekte Methoden zum Ausschluß von viruskontaminierten Plasmaspenden zur Verfügung stehen und besteht die Möglichkeit, daß das Ausgangsmaterial so stark kontaminiert ist, daß die ansonsten erfolgreich anwendbaren Virusinaktivierungs- und Virusabreicherungsmethoden nicht mehr genügen, um virussichere Endprodukte herzustellen.

Die menschliche infektiöse Dosis ist für HIV, HCV und HBV nicht bekannt und derzeit nicht bestimmbar. Die Bestimmung der infektiösen Dosis in Gewebekulturen (TCID₅₀: tissue culture infectious dose) oder im Tiermodell (CID₅₀: chimpanzee infectious dose) gibt daher nur eine Annäherung der humanen infektiösen Dosis an. Dazu kommt noch, daß in einigen Fällen Menschen trotz einer Exposition mit HIV, HCV oder HBV nicht infiziert werden. Eine humane infektiöse Dosis eines solchen Virus ist daher nicht unbedingt infektiös.

Piatak et al. (Science 1993, 259:1749-1754) ermittelten die TCID₅₀ von HIV mit 1 TCID₅₀/10⁴ Kopien HIV RNA. Shimizu, Y.K. et al. (PNAS. Natl. Acad. Sci. USA 1993, 90: 6037-6041) bestimmten die CID₅₀ eines HCV-Stammes mit 1 CID₅₀/1 Kopie RNA.

Von Eder et al. (The Role of the Chimpanzee in Research, Symp. Vienna 1992, 156-165) konnte gezeigt werden, daß 20 Kopien HBV DNA/ml Plasma einer CID₅₀ von 1 entsprechen.

Es ist bei einem Plasmapool oder anderen Plasmaausgangsmaterialien insbesondere darauf zu achten, daß die Virusbelastung so gering wie möglich ist, sodaß gegebenenfalls durch zusätzliche Virusinaktivierungsschritte bzw. Maßnahmen zur Abreicherung von Viren die Virusbelastung zumindest auf unterhalb der infektiösen Dosis gesenkt werden kann. Wünschenswert wäre eine Virusbelastung in einem Ausgangsmaterial, bei der die Kopienzahl der viralen Nukleinsäure schon unterhalb der infektiösen Dosis für Schimpansen liegt.

Die Aufgabe der vorliegenden Erfindung besteht darin, Verfahren zur Herstellung eines Arzneimittels enthaltend ein oder mehrere Plasmaderivate zur Verfügung zu stellen, welches die Gefahr der Kontamination mit vermehrungsfähigen haematogenen Viren aufgrund von übermäßiger Kontamination bestimmter Ausgangschargen nicht mehr aufweisen kann.

Dabei besteht die engere Aufgabe darin, ein qualitätsgesichertes Ausgangsmaterial oder Zwischenprodukt zur Verfügung zu stellen, deren Virusbelastung einen vorgegebenen Höchstwert nicht überschreiten darf.

Die Aufgabe wird erfindungsgemäß durch die Verfahren gemäß Anspruch 1-8 gelöst.

Die Lösung der Aufgabe beinhaltet die Feststellung des Ausmaßes der Kontamination bzw. der Virusbelastung im Ausgangsmaterial, welche Virusbelastung durch ein Verfahren zur Inaktivierung oder Abreicherung der Viren nicht mehr eliminiert werden kann. Es wird daher das Problem gelöst, in einem Ausgangsmaterial, die potentielle Viruskontamination festzustellen und jene Ausgangsmaterialien so lange nicht weiter zu verarbeiten bzw. von der Verarbeitung auszuschließen, als dieser hohe Kontaminationsgrad besteht.

Dafür soll jedes verwendete Ausgangsmaterial für die Plasmaderivate und eventuell auch die bei der Herstellung der Plasmaderivate anfallenden Zwischenprodukte, die einem Virusabreicherungs- oder Virusinaktivierungsschritt unterzogen werden sollen, und fertige Plasmaderivate so untersucht und behandelt werden, daß in gesicherter Weise gewährleistet wird, daß derartige aus Blutplasma hergestellte Arzneimittel auch nicht mehr sporadisch haematogene vermehrungsfähige Viren übertragen können. Dabei soll das Ausgangsmaterial mit Hilfe ausgewählter Amplifikationstechniken untersucht werden, um die obere Grenze einer möglichen Virusbelastung für ein Ausgangsmaterial bzw. Zwischenprodukt festzustellen, das noch mindestens einem wesentlichen Virusinaktivierungs- bzw. Virusabreicherungsschritt unterzogen wird, um zu einem Arzneimittel zu gelangen, das keine Übertragung von haematogenen Viren zur Folge hat.

Die bei der Bestimmung der Gemonäquivalente zu detektierenden Zielnukleinsäuren können durch verschiedene Polymerase-Ketten-Reaktionen (PCR) bzw. spezielle Arten der PCR als Nachweis bzw. Bestimmungsverfahren entsprechend der vorliegenden Erfindung im Blut, im Plasma, im Serum oder Plasmafraktionen amplifiziert werden. Für den Nachweis von spezifischen viralen Genomsequenzen muß der Amplifikationsprozeß selektiv für die zu amplifizierende Nukleinsäuresequenz sein. Die Amplifikation von Nukleinsäuren in den erfindungsgemäßen Plasmapools kann durch eine Reihe von in der Literatur beschriebenen Amplifikationsprozessen erfolgen.

Die Amplifikation spezifischer viraler Genomsequenzen erfordert die Kenntnis des Genoms der einzelnen doch sehr unterschiedlichen Viren, um sie zu vervielfältigen und detektierbar zu machen.

Verschiedene Amplifikationsverfahren für Nukleinsäuren basieren auf der PCR. Das PCR-Amplifikationsverfahren wurde erstmals 1983 von Mullis et al. (US 4,683,195 und US 4,683,202) beschrieben. Virale Genomsequenzen können ebenfalls durch "nested PCR" (Mullis et al Methods Enzymol. 1987, 155: 335-350) oder durch RT-PCR (Powell, L.M., et al. Cell 1987, 50:831-840; Kawasaki, et al. Proc. Natl. Acad. Sci. USA 1988, 85:5698-5702) amplifiziert werden.

Für die Amplifikation und den Nachweis von RNA muß die RNA erst in die DNA transkribiert werden. Dieses Verfahren wird in der WO 91/09944 unter Verwendung der Reversen-Transkriptase als sogenannte RT-PCR beschrieben.

Die Analyse der Amplifikationsprodukte kann durch Verwendung von markierten Nukleotiden oder Oligonukleotid-Primern beim Elongationsprozeß und der anschließenden Hybridisierung oder gelelektrophoretischen Auftrennung der Produkte erfolgen.

Alternativ-Verfahren zur PCR wurden ebenfalls beschrieben.

Eines dieser Verfahren zur Nukleinsäureamplifikation ist die LCR (Ligase chain reaction) entsprechend der EP 0 320 308, EP 0 336 731 und Wu und Wallace (Genomics 1989, 4: 560-569).

Andere enzymatische Prozesse sind die NASBA (nucleic acid sequence based amplification), die 3SR (self-sustained sequence replication) entsprechend EP 0 310 229 und Guatelli, J.C. et al. (Proc. Natl. Acad. Sci. USA 1990, 87: 1874-1878) oder die TAS (transcription based amplification System) entsprechend EP 0 373 960 und Kwoh, D.Y. et al. (Proc. Natl. Acad. Sci. USA 1989, 86: 1173-1177). In diesen Verfahren wird eine Reihe von Enzymen entweder gleichzeitig oder schrittweise bei der Amplifikation verwendet, wie z.B. eine DNA-Polymerase oder eine RNA-Polymerase.

Andere Amplifikationsprozesse basieren auf der Verwendung der Replikase des RNA-Bakteriophagen Qβ entsprechend EP 0 361 983 und Lizardi et al. (TIBTECH 1991, 9: 53-58).

Ein weiteres Verfahren beschreibt die Signal-Amplifikation durch verzweigte DNA-Oligonukleotide anstelle der extrahierten Nukleinsäure (branched DNA signal amplification) entsprechend Urdea, M.S. et al. Nucleic Acids Res. Symp. Ser. 24 Oxford 1991, Pachl, C.A. et al. XXXII Interscience Conference on Antimicrobial Agents and Chemotherapy. Anaheim, October 1992 (abstract 1247).

In der EP 0 590 327 A2 ist ein Analysenverfahren für Blutproben beschrieben, bei dem die Bestimmung viraler RNA oder DNA von beispielsweise Hepatitis C-Virus, HIV oder Hepatitis B-Virus vorgesehen ist. Die Nachweisgrenze für einen Test zur Bestimmung von Hepatitis B-Virus-Genom beträgt 1.500 Kopien (150.000 Kopien/ml), wenn die Auswertung elektrophoretisch nach dem Färben des Gels erfolgt. Die Blutproben sind z.T. getrocknete Blutflecken. Nach erfolgter Analyse ist keine weitere Verarbeitung der Proben vorgesehen.

Verfahren zur Herstellung von Hyperimmunglobulin-Präparaten gegen bestimmte Viren gehen von Blut- bzw. Plasma aus, das von Spendern stammt, die aktiv immunisiert sind oder eine Erkrankung bereits überstanden haben und daher eine protektive Immunität aufweisen. Plasmaspender, die gegen pathogene Viren geimpft worden sind, enthalten in ihrem Blut einen entsprechenden Antikörpertiter. Diese Antikörper sind im Gegensatz zu Antikörper, die auf eine Virämie hinweisen, wie z.B. HIV-Antikörper, erwünscht.

Das zur Herstellung der Arzneimittel verwendete erfindungsgemäß qualitätsgesicherte Ausgangsmaterial bzw. Zwischenprodukt enthält also entweder einen Überschuß von virusneutralisierenden Antikörpern gegen ein interessierendes Virus oder keine nachweisbaren oder unter einem Grenzwert liegende Menge Genome oder Genomfragmente von möglicherweise vorkommenden haematogenen vermehrungsfähigen Viren.

Die Plasmaderivate können im Arzneimittel sowohl als Wirksubstanz als auch als Hilfsstoff enthalten sein. Wirksubstanzen umfassen beispielsweise Gerinnungsfaktoren, Immunglobuline, Fibrinolysefaktoren, Enzym-Inhibitoren oder andere im Plasma vorhandene Enzyme bzw. Zymogene, sowie Mischungen davon. Hilfsstoffe umfassen Albumin oder andere Stabilisatoren, verschiedene Inhibitoren, Cofaktoren, etc.

Als Ausgangsmaterial ist prinzipiell jedes Material anzusehen, welches am Anfang eines bestimmten Herstellungsprozesses steht, beispielsweise ein Plasmapool, ein Kryopoor-Plasma oder eine Cohn II+III-Paste. Eine Reihe von möglichen Ausgangsmaterialien sind in einem Artikel von Heide et al zitiert ("Plasma Protein Fractionation" in "The Plasma Proteins", Frank W. Putnam, ed. Academic Press New York, San Francisco, London 1977, S. 545-597).

Zwischenprodukte sind vom Ausgangsmaterial in einem bestimmten Herstellungsprozeß abgeleitete Produkte, welche durch die vorgesehenen Herstellungsschritte gewonnen werden, z.B. ein Prothrombinkomplex bei der Herstellung von Faktor IX mit Plasma oder Kryopoor-Plasma als Ausgangsmaterial.

Aus dem Ausgangsmateiral wird durch den produktspezifischen Herstellungsprozeß gegebenenfalls über Zwischenprodukte ein fertiges Plasmaderivat hergestellt, welches mit routinemäßigen Konfektionierungsmaßnahmen zu einem Arzneimittel aufgearbeitet wird. Bei dieser Aufarbeitung können natürlich auch zwei oder mehrere fertige Plasmaderivate miteinander vereinigt werden.

Bevorzugterweise besteht die nicht vorhandene Virusbelastung bzw. die den definierten Grenzwert nicht überschreitende Virusbelastung für mindestens zwei Viren, vorzugsweise ausgewählt aus der Gruppe HIV, HAV, HBV und HCV, insbesondere HIV und HCV.

Selbstverständlich ist die vorliegende Erfindung nicht auf die oben erwähnten Viren beschränkt, sondern die nicht vorhandene bzw. einen definierten Grenzwert nicht überschreitende Virusbelastung kann für jedes haematogene Virus festgestellt werden, also z.B. auch für Parvovirus, Hepatitis non A non B Virus und für Viren, welche neu entdeckt werden, durch Blut oder aus Blut gewonnenen Produkten übertragbar sind und sich als risikoreich für den Menschen herausstellen. Vorzugsweise wird die nicht vorhandene bzw. einen definierten Grenzwert nicht überschreitende Virusbelastung für alle bekannten haematogenen Viren festgestellt, soferne die Anwesenheit dieser Viren im Arzneimittel eine Gefahr für den Patienten darstellen würde.

Der Grenzwert der zulässigen Belastung an vermehrungsfähigen haematogenen Viren, welcher jedenfalls unterschritten werden sollte, richtet sich in erster Linie nach dem Potential der Nachweisreaktion und nach dem Aufwand, welcher bei der Beurteilung des Ausgangsmaterials bzw. Zwischenprodukts zweckmäßig ist. Die Nachweisgrenze hängt beispielsweise vom Volumen an Probe ab, welche zur Nachweisreaktion verwendet wird. Wenn zum Beispiel in 20 µl Probe einer Einzelspende kein virales Genomäquivalent nachgewiesen werden kann und die eingesetzte Nachweismethode in der Lage ist, ein einziges Genomäquivalent in der Probe nachzuweisen, so ist daraus zu schließen, daß die maximale Belastung der Einzelspende unter 50 Genomäquivalenten pro ml Plasma liegt. Werden bei negativem Nachweisergebnis weitere 20 µl Probe getestet und erneut kein virales Genomäquivalent nachgeweisen, so kann daraus geschlossen werden, daß die maximale Belastung der Einzelspende unter 25 Genomäquivalente pro ml Plasma liegt.

Als gut für die praktische Arbeit geeignete Grenzwerte der zulässigen Belastung an vermehrungsfähigen haematogenen Viren haben sich Werte von maximal 500 Genomäquivalenten, insbesondere maximal 200 Genomäquivalente, bevorzugt 100 Genomäquivalente, besonders bevorzugt 50 Genomäquivalente, pro ml Ausgangsmaterial bzw. Zwischenprodukt herausgestellt.

Bei einem bevorzugten erfindungsgemäß gesicherten Ausgangsmaterial bzw. Zwischenprodukt wird eine Virusinaktivierung bzw. Virusabreiherung mittels mindestens eines Verfahrens mit einem Reduktionsaktor von mindestens 4 bei der Herstellung des Arzneimittels aus diesem qualitätsgesicherten Ausgangsmaterial bzw. Zwischenprodukt, erreicht.

Das erfindungsgemäß herstellbare Plasmaprotein-hältige Arzneimittel hat den Vorteil, daß aufgrund der gesicherten limitierten Virusbelastung an einem oder mehreren vermehrungsfähigen haematogenen Viren im Ausgangsmateiral des Plasmaproteins ein proteinschonendes Verfahren zur Virusinaktivierung bzw. Virusabreicherung ausreicht, um das plasmaprotein in virussicherer Form zu erhalten. Vorzugsweise wird ein proteinschonendes Verfahren mit einem Reduktionsfaktor von max. 4 vorgenommen, um eine gegebenenfalls vorhandene Virusbelastung an vermehrungsfähigen haematogenen Viren zu inaktivieren bzw. zu eliminieren. Ein proteinschonendes Verfahren ermöglicht die Erhaltung der Aktivität und Integrität eines Plasmaproteins in einem größtmöglichen Ausmaß. Als proteinschonend wird ein Verfahren angesehen, bei welchem eine Erhaltung der (spezifischen) Aktivität des zu gewinnenden Proteins von 50 % oder mehr, vorzugsweise 80 % oder mehr, erzielt wird. Es reicht sogar eine einzige Virusinaktivierung bzw. Virusabreicherung im Zuge des Herstellungsverfahrens für das erfindungsgemäße Arzneimittel aus, um gegebenenfalls vorhandene Viren vollständig zu inaktivieren bzw. zu eliminieren.

Es hat sich gezeigt, daß bei einem zur Herstellung von Plasmaderivaten bzw. Arzneimitteln verwendeten Ausgangsmaterial bzw. Zwischenprodukt, dem ein Testvirus zugesetzt worden ist, die Virusbelastung vor einem vorgesehenen Virusinaktivierungsschritt durch eine Virusabreicherung stark herabgesetzt werden kann (z.B. EP-A-0 506 651). Die Abreicherung kann durch bekannte Verfahren, wie Nanofiltration, Fällungsreaktionen, Dialyse, Zentrifugation, chromatographische Reinigungsschritte, etc. erfolgen. Zur Inaktivierung von Viren sind eine Reihe von physikalischen, chemischen oder chemisch/physikalischen Methoden bekannt, die beispielsweise eine Hitzebehandlung, z.B. gemäß der EP-A-159 311 oder EP-A-0 637 451, eine Hydrolasebehandlung gemäß der EP-A-0 247 998, eine Strahlenbehandlung oder eine Behandlung mit organischen Lösungsmitteln und/oder Tensiden, z.B. gemäß der EP-A-0 131 740 umfassen. Weitere geeignete Virusinaktivierungsschritte bei der Herstellung von Plasmafraktionen und Arzneimitteln sind in der EP-A-0 506 651 oder in der WO94/13329 beschrieben. Eine Analyse von verschiedenen Inaktivierungsmaßnahmen findet sich in dem Dokument Eur.J. Epidermal. 3 (1987), 103-118; zum Reduktionsfaktor liegt die Richtlinie ECIII/8115/89-EN der Kommission der EG vor.

Die erfindungsgemäßen Maßnahmen ermöglichen ein gepooltes Ausgangsmaterial, insbesondere einen Plasmapool, bzw. ein Zwischenprodukt von hervorragender Qualität mit erhöhter Sicherheit.

Vorzugsweise werden diejenigen Plasmaspender zur Herstellung des erfindungsgemäß qualitätsgesicherten Plasmapools ausgewählt, die aktiv gegen ein oder mehrere der Viren immunisiert oder immun sind, die also eine protektive Immunität aufweisen, beispielsweise durch eine entsprechende Impfung. Die Plasmaspender sind vorzugsweise gegen ein Hepatitis-Virus, insbesondere Hepatitis-A-Virus, geimpft, um eine entsprechende Infektiösität des Plasmas auszuschließen.

Wenn Plasma das Ausgangsmaterial ist, bestehen bevorzugte Ausführungsformen der erfindungsgemäßen Qualitätssicherung darin, daß
- die Bestimmung der Genomäquivalente sämtlicher Viren der Gruppe HIV, HBV, HCV, Parvovirus und HAV,
- die Bestimmung der Genomäquivalente sämtlicher Viren der Gruppe HIV, HBV, HCV und Parvovirus, sowie die Feststellung eines Überschusses von HAV-Antikörpern,
- die Bestimmung der Genomäquivalente sämtlicher Viren der Gruppe HIV, HBV und HCV, sowie die Feststellung eines Überschusses von HAV- und Parvovirus-Antikörpern,
- die Bestimmung der Genomäquivalente sämtlicher Viren der Gruppe HIV, HBV und HCV, sowie die Feststellung eines Überschusses von HAV-Antikörpern,
- die Bestimmung der Genomäquivalente sämtlicher Viren der Gruppe HIV, HBV und HCV, sowie die Feststellung eines Überschusses von Parvovirus-Antikörpern,
- die Bestimmung der Genomäquivalente sämtlicher Viren der Gruppe HIV, HBV und HCV,
- die Bestimmung der Genomäquivalente sämtlicher Viren der Gruppe HIV und HCV, oder
- die Bestimmung der Genomäquivalente sämtlicher Viren der Gruppe HIV und HCV, sowie die Feststellung eines Überschusses von MBV-Antikörpern,
erfolgt.

Der erfindungsgemäß qualitätsgesicherte Plasmapool ist beispielsweise auch erhältlich durch die Auswahl von Plasmaspendern, die gegen Hepatitis A- und Hepatitis B-Virus geimpft sind und die damit aufgrund ihrer antiinfektiösen Immunität einen Antikörpertiter in ihrem Blut aufweisen können. Diese Antikörper sind infolge einer aktiven Immunisierung gebildet worden und daher nicht indikativ für eine Virämie. Die Plasmaspenden werden einzeln auf die Abwesenheit von Markern untersucht, die auf eine entsprechende Virämie hinweisen. Dazu wird beispielsweise ein Test zur Bestimmung von Hepatitis C-Virus- und gegebenenfalls HIV-Antikörpern mit Hilfe eines validierten ELISA-Testsystems durchgeführt. Als Marker für Hepatitis C-Virus gelten auch bestimmte Leberwerte, wie z.B. ALT- und GPD-Werte. Falls sich die Abwesenheit der viralen Marker im Spenderplasma bestätigt, wird als weiteres Auswahlkriterium der Nachweis für die Abwesenheit eines Genoms oder Genomfragmentes von AIDS-Viren (HIV-1) und gegebenenfalls Hepatitis C-Virus in einem Plasmapool der Einzelplasmaspenden erbracht. Diese Vorgangsweise empfiehlt sich trotz Bestätigung, daß diese Plasmaspende keine Antikörper gegen HIV enthält, welche indikativ für eine virale Infektion sind. Die Zeit von der Infektion mit einem HIV-Virus bis zur möglichen Detektion von entsprechenden Antikörpern kann sich gerade bei diesen Viren über Monate erstrecken. Ein Plasmapool, der lediglich auf Abwesenheit von HIV-Antikörper getestet wurde, kann daher nicht nachweislich als kontrolliertes Plasmapool zur Verfügung gestellt werden, der keine oder unter einem definierten Grenzwert liegenden vermehrungsfähigen, infektiösen Viren enthält.

Eine der erfindungsgemäßen Maßnahmen zur Sicherung der limitierten Belastung an vermehrungsfähigen haematogenen Viren umfaßt neben dem quantifizierbaren, kontrollierten und nicht-inhibierten Verfahren zum Nachweis bzw. zur Bestimmung von Nukleinsäuren den Nachweis von virusneutralisierenden Antikörpern im erfindungsgemäßen Plasmapool. Zur Bestimmung des Antikörpertiters sind ebenfalls validierte Testsysteme einzusetzen, beispielsweise entsprechende Enzymimmuntests. Die für das Verfahren eingesetzten Proben können gegebenenfalls lyophilisiert und anschließend rekonstitutiert sein.

Als Ausgangsmaterial kommen für diese Erfindung außer Plasmapools auch Kryopräzipitate oder andere frühe Intermediärprodukte in Frage. Frühe Intermediärprodukte sind solche, die aus menschlichem Plasma bei der Erzeugung von Arzneimitteln gewonnen werden, die noch einer Virusabreicherung oder -inaktivierung unterzogen werden. Auch das zum Plasmapool hier ausgesagte gilt - soweit möglich - erfindungsgemäß auch für andere Ausgangsmaterialien, insbesondere die Möglichkeit der Vereinigung gleichartig gesicherter kleinerer Mengen zu größeren. Erfindungsgemäß ist daher das Vermischen von gleichartigen qualitätsgesicherten Ausgangsmaterialien bzw. Zwischenprodukten zu einem größeren qualitätsgesicherten Ausgangsmaterial- bzw. Zwischenproduktpool vorgesehen.

Derart hohe Qualitätskriterien für ein Ausgangsmaterial bzw. Zwischenprodukt wurden bislang nicht erfüllt. Einerseits wurde es nicht für notwendig erachtet, eine Virämie bedingt durch einen mit Virus belasteten Plasmapool vollkommen auszuschließen. Plasmapools werden vor allem zur Herstellung von Plasmaderivaten verwendet, also von pharmazeutischen Produkten auf Basis von Plasmaproteinen. Bei dieser Herstellung muß jedenfalls eine zusätzliche Maßnahme zur Inaktivierung und/oder Abreicherung von potentiell vorhandenen Viren vorgenommen werden, was das Risiko einer Übertragung der pathogenen Viren durch die fertigen Produkte um ein Vielfaches reduziert, sodaß die vorgeschriebenen gemäß dem Stand der Technik üblichen Untersuchungen als ausreichend angesehen werden.

Erfindungsgemäß kommen nur solche Ausgangsmaterialien bzw. Zwischenprodukte für die weitere Produktaufbereitung zum Einsatz, die einen Überschuß von virusneutralisierenden Antikörpern oder gesicherte limitierte Belastung an viralen Genomsequenzen aufweisen. Ausgangsmaterial, welches den erfindungsgemäßen Anforderungen nicht genügt, ist zwar nicht für die Weiterverarbeitung zu Arzneimitteln im Rahmen der vorliegenden Erfindung geeignet, kann aber trotz allem nicht bewiesenermaßen als infektiös bezeichnet werden, da die mit einer Nukleinsäure-Amplifikationsmethode festgestellte Menge an Genomäquivalenten nur die Obergrenze für die Belastung an vermehrungsfähigen Viren angibt. Die festgestellte Menge an Genomäquivalenten gibt daher nur dann den "wahren Wert" der Belastung an vermehrungsfähigen Viren an, wenn alle Genomäquivalente von aktiven Viren stammen.

Aus mehreren erfindungsgemäß gesicherten Ausgangsmaterialien kann durch Vermischen eine größere Menge erhalten werden, welche denselben Sicherheitskriterien entspricht.

So kann im Falle eines Plasmapools dieser als Smallpool zur Verfügung gestellt werden, der aus etwa 2 bis 20 Einzelplasmaspenden zusammengesetzt ist. Mindestens 10 Smallpools können zu einem Minipool von etwa 20 bis 200, vorzugsweise etwa 200, Einzelplasmaspenden kombiniert werden. Die nächste Größenordnung ist ein Makropool mit einer Größe von etwa 200 bis 2000, vorzugsweise etwa 2000, Einzelplasmaspenden, der gegebenenfalls durch Vermischen von mindestens 10 Minipools hergestellt ist. Mehrere Makropools können zu einem Multimakropool bis zu einer Größe von 200 000 Einzelspenden zusammengesetzt werden.

Der Nachweis für virale Genome oder Genomfragmente kann erfindungsgemäß für jede dieser Poolgrößen erbracht werden. Durch das Testen der Smallpools und die anschließende Kombination der kontrollierten Smallpools zu einem größeren Pool, beispielsweise einem Minipool, wird eine bisher noch nie beschriebene Qualität auch von einem größeren Pool erhalten.

Eine besondere Ausführungsform der Erfindung besteht daher in der Herstellung eines Plasmapools der durch Vermischen von Minipools, die vorzugsweise aus etwa 200 Einzelspenden bestehen, zu einem Makropool, der vorzugsweise aus etwa 2000 Einzelspenden besteht, gewonnen ist.

Eine weitere bevorzugte Ausführungsform besteht in der Herstellung eines Plasmapools, welcher dadurch gekennzeichnet ist, daß ein Makropool durch Vermischen mit einer beliebigen Anzahl weiterer Makropools zu einem Multimakropool bis zu 200.000 Einzelspenden vereinigt wird.

Noch eine bevorzugte Ausführungsform besteht in der Herstellung eines Plasmapools bei welchem die Minipools durch Vermischen von Smallpools, die aus zwei bis etwa 20 Einzelspenden bestehen, gewonnen werden.

Bei der Wahl eines Ausgangsmaterials zur Fraktionierung von Blutplasma zur Herstellung von Plasmafraktionen bzw. fertigen Plasmaderivaten und Arzneimitteln ist eine relativ große Menge aus Gründen der Wirtschaftlichkeit vorzuziehen. Bislang stand aber noch kein Plasmapool der Größenordnung eines Makropools oder eines Multimakropools zur Verfügung, der durch den Nachweis von viralen Genomen oder Genomfragmenten als nicht-virusbelastet gelten konnte. Es genügt nämlich nicht, den Nachweis allein für einen Multimakropool zu erbringen, da durch das Zusammenmischen von mehr als 2000 Einzelplasmaspenden ein zu großer Verdünnungseffekt auftritt und die Anzahl der zu detektierenden Genome sehr häufig kleiner als die Nachweisgrenze eines Testverfahrens ist. Durch das Testen von kleinen Untereinheiten und das anschließende Kombinieren zu größeren Einheiten wird dieses Problem der Nachweisbarkeit überwunden und eine wirtschaftlich interessante Menge erhalten.

Das erfindungsgemäß qualitätsgesicherte Ausgangsmaterial eignet sich hervorragend zur Herstellung von Fraktionen enthaltend Plasmaproteine als Zwischenprodukte bzw. zur Herstellung von fertigen Arzneimitteln.

Die für das Ausgangsmaterial, insbesondere Plasmapools vorgesehenen Maßnahmen sind selbstverständlich analog bei Plasmafraktionen aller Art anwendbar. Gemäß einem weiteren Aspekt betrifft daher die vorliegende Erfindung auch die Herstellung qualitätsgesicherte fertige Plasmaderivate mit durch Verwendung von qualitätsgesichertem Ausgangsmaterial gesicherter limitierter Belastung an vermehrungsfähigen haematogenen Viren.

Weiters betrifft die vorliegende Erfindung ein Verfahren zur Herstellung eines Arzneimittels enthaltend ein oder mehrere Plasmaderivate aus einem qualitätsgesicherten Ausgangsmaterial bzw. aus einem bei der Herstellung der Plasmaderivate anfallenden qualitätsgesicherten Zwischenprodukt, wobei das qualitätsgesicherte Ausgangsmaterial bzw. Zwischenprodukt keine oder eine einen definierten Grenzwert nicht überschreitende Virusbelastung an einem oder mehreren vermehrungsfähigen haematogenen Viren aufweist und der definierte Grenzwert im Falle der Bestimmung der Genomäquivalente des jeweiligen Virus durch ein quantifizierbares, kontrolliertes und nicht-inhibiertes Verfahren zum Nachweis bzw. zur Bestimmung von Nukleinsäuren ausgewählt ist nach einem oder mehreren der folgenden Kriterien:
- Nachweisgrenze (des) der Nukleinsäure-Amplifizierungsverfah-. ren(s) von mindestens 10 000 Kopien ausgewählter Nukleinsäuresequenzen;
- bestimmte Anzahl an Genomäquivalenten, insbesondere eine Anzahl unter 500 Genomäquivalenten, vorzugsweise unter 200 Genomäquivalenten, noch bevorzugter unter 100 Genomäquivalenten, besonders bevorzugt unter 50 Genomäquivalenten, pro ml Ausgangsmaterial bzw. Zwischenprodukt;
Andere zulässige Auswahlkriterien bezüglich der Grenzwerte sind erfindungsgemäß
- ein mit einem Zellkulturtest ermittelter Grenzwert, vorzugsweise eine TCID₅₀ pro ml, bzw. die in dieser Menge enthaltenen Genomäquivalente,
- ein mit einem Tiermodell ermittelter Grenzwert, vorzugsweise eine CID₅₀ pro ml, bzw. die in dieser Menge enthaltenen Genomäquivalente

Diese Grenzwerte sind wegen ihrer praktischen Relevanz (abhängig vom Nachweispotential einer Untersuchungsmethode) und ihrer biologischen Relevanz (speziell die TCID- und CID-Werte) bevorzugt.

Die hochsensitive Methode der PCR hat den Nachteil, daß schon bestimmte geringste Verunreinigungen, die in das Testmaterial gelangen können, ebenfalls amplifiziert werden, so daß es zu falsch positiven Ergebnissen kommen kann.

Eine bevorzugte Ausführungsform des erfindungsgemäßen Verfahrens ist dadurch gekennzeichnet, daß mehr als ein Nachweis- bzw. Bestimmungssystem für Nukleinsäuren eingesetzt wird, wobei die Nachweis- bzw. Bestimmungssysteme vorzugsweise derart ausgewählt sind, daß das eine System falsch positive und ein anderes System falsch negative Resultate ausschließt.

Vorzugsweise werden mindestens zwei unterschiedliche PCR-Methoden eingesetzt, wobei mindestens eine Methode zum Screening und mindestens eine Methode zur Bestätigung (Konfirmation) dient.

Bei der DTS-PCR (Dual Targeting-Southern Blot-PCR) erfolgt nach Amplifikation der extrahierten Nukleinsäuren eine Auftrennung der synthetisierten Nukleinsäuren durch Agarose-Gelelektrophorese und anschließendem Southern-Blot nach Hybridisierung mit einer Digoxigenin (DIG)-markierten Sonde. Die Auswertung erfolgt über eine densitometrische Bestimmung der Bandenintensität. Zum Ausschluß von falsch negativen Ergebnissen, die durch ein Mispriming zwischen PCR-Primern und Template aufgrund von Sequenzheterogenität verursacht sein kann, werden die Nukleinsäure-Extrakte in einer zweiten PCR mit weiteren, von dem ersten Primerpaar verschiedenen, Primern nochmals amplifiziert und analysiert. Durch die Zugabe einer synthetischen Nukleinsäure als internen Standard wird die DTS-PCR kontrolliert und falsch negative Resultate ausgeschlossen.

Um beispielsweise die in der DTS-PCR erhaltenen Resultate zusätzlich zu verifizieren, wird vorzugsweise als weitere Methode die LIF-PCR (Laser-induzierte Fluoreszenz-PCR) eingesetzt.

Durch Verwendung von nichtradioaktiv-markierten Primern (bevorzugt werden Fluoreszenzfarbstoff-markierte Primer eingesetzt) können die PCR-Produkte durch LIF-PCR nachgewiesen werden. Die Detektion der Amplifikationsprodukte kann aber auch über den Einbau von nichtradioaktiv-markierten Nukleotid-Analogen während der Elongationsreaktion in die neusynthetisierten Nukleinsäuren vereinfacht werden. Bei der LIF-PCR erfolgt die Amplifikation der Nukleinsäure in Gegenwart von fluoreszenzmarkierten Primern und anschließender Auftrennung der Amplifikationsprodukte durch PAGE. Bei jedem Probenansatz der LIF-PCR werden zusätzlich Negativ- und Positiv-Kontrollen mitgeführt. Der Nachweis und die Quantifizierung der PCR-Produkte erfolgen mittels eines Genescanners. Mit Hilfe der internen Doppelstandardisierung des LIF-PCR werden insbesondere falsch negative Ergebnisse ausgeschlossen und positive Ergebnisse abgesichert.

Falsch positive bzw. falsch negative Ergebnisse können erfindungsgemäß durch den Einsatz von Positiv-und Negativ-Kontrollen ausgeschlossen werden. Die geschilderte Kombination zweier verschiedener PCR-Methoden, insbesondere der DTS-PCR und der Laser-induzierten Fluoreszenz-PCR (LIF-PCR) ermöglicht die Prüfung großer Probenzahlen in der Routinekontrolle und somit den Ausschluß primär falsch negativer Resultate und die Vermeidung falsch positiver Ergebnisse.

Ein zusätzlicher Aspekt der Erfindung liegt darin, daß eine Aufkonzentrierung der Testproben vorgenommen wird und dadurch noch geringere Belastungen an viralen Genomsequenzen detektiert werden können. Die Empfindlichkeit des erfindungsgemäßen Verfahrens kann durch zusätzliche Maßnahmen durch Konzentrierung der Plasmaproben oder der enthaltenen Genomäquivalente gesteigert werden, wobei eine Erhöhung der Sensitivität um das 10fache bis 100fache bevorzugt ist.

Bei einer bevorzugten Ausführungsform des erfindungsgemäßen Verfahrens werden daher die Proben aus den Ausgangsmaterialien (z.B. Einzelspenden oder Probenpools) bzw. Zwischenprodukt bzw. die darin enthaltenen Genomäquivalente konzentriert, und zwar vorzugsweise durch Lyophilisation, Adsorption oder Zentrifugation.

Erfindungsgemäß wird eine Methode herangezogen, die ein quantifizierbares, kontrolliertes und nicht-inhibiertes Verfahren zum Nachweis bzw. zur Bestimmung von Genomsequenzen ermöglicht. Bei der Bestimmung der limitierten Belastung an vermehrungsfähigen Viren des Plasmapools durch den Nachweis von viralen Genomen oder Genomsequenzen kann es aber vorkommen, daß gewisse Inhibitoren der Nachweismethode in den Proben vorhanden sind, sodaß nicht von vornherein eine nicht-inhibierte Amplifikation möglich ist.

Es hat sich nämlich herausgestellt, daß die Virusbelastung eines Plasmapools bestimmt mit einer PCR-Methode durchaus unterschätzt werden kann. Im Plasmapool können Faktoren anwesend sein, die die Empfindlichkeit eines PCR-Bestimmungsverfahrens erheblich beeinträchtigen können (Nucleic Acids Research 16 (21), 10355 (1988)). Gegebenenfalls vorhandene Inhibitoren der PCR-Reaktion werden bei dem erfindungsgemäßen Verfahren vor der Nachweisreaktion entfernt und durch eine Verwendung eines Genomstandards während der Nachweisreaktion ausgeschlossen. Falls der interne Standard mit der entsprechenden Rate im Pool amplifizierbar und detektierbar ist, ist die Anwesenheit von solchen Inhibitoren ausgeschlossen.

Es ist bekannt, daß im Plasma vorkommende Substanzen, wie Heparin, hohe Salzkonzentrationen und Polyethylenglycol die PCR inhibieren können (BioTechniques 9(2),166 (1990) und Journal of Clinical Microbiology 29(4) 676-679 (1991)). .

Das erfindungsgemäße Verfahren ist daher dadurch gekennzeichnet, daß gegebenenfalls vorhandene Inhibitoren der Amplifikation in den Proben aus den Einzelspenden oder in den Probenpools entfernt oder abgereichert werden.

Diese zusätzlichen Verfahren zum Entfernen oder zur Neutralisation von Amplifikations-Inhibitoren im Blut, Plasma oder Serum umfassen z.B. die Ultrazentrifugation der Proben und das Dekantieren des Überstandes mit darin enthaltenen Inhibitoren und die Extraktion der Nukleinsäure, sowie die Behandlung der Proben mit Heparinase, Polyaminen oder die Vorreinigung der Nukleinsäuren mittels HPLC. Durch die erhöhte Reinheit der Nukleinsäuren wird eine uneingeschränkte Amplifikation der Nukleinsäure gewährleistet.

Gemäß einer bevorzugten Verfahrensvariante werden für mehrere Viren spezifische Primer-Paare beim Amplifikationstest eingesetzt, so daß die Anwesenheit mehrerer Virusarten gleichzeitig untersucht werden kann. Bei einem besonders bevorzugten Test werden zwei oder mehrere Viren, ausgewählt aus der Gruppe HIV, HAV, HBV und HCV, untersucht.

Insbesondere RNA-Viren unterliegen einer großen Mütationsrate, wodurch sehr schnell Varianten oder Subtypen eines bekannten Stammes auftreten können, die sich in ihrer Genomsequenz von den Wildtypesequenzen mehr oder weniger stark unterscheiden. Bei einer empfindlichen Amplifikationsmethode wie z.B. der PCR ist jedoch für den Nachweis und die quantitative Bestimmung sehr geringer Kopienzahl einer viralen Genomsequenz eine besonders hohe Effizienz der Amplifikation Voraussetzung, um die in einer Probe tatsächlich vorhandenen Kopien festzustellen. Eine ungenügende Paarung der Primer mit dem Template verringert deren Effizienz.
Eine entsprechende Sorgfalt bei der Auswahl der Primer muß daher gewährleistet werden.

Bevorzugte Primer-Paare werden so ausgewählt, daß sie für konservierte Genomsequenzen der einzelnen zu untersuchenden haematogenen Viren kodieren, wobei die Eignung der Primer an entsprechenden Proben eines repräsentativen Probequerschnitts der zu untersuchenden Viren festgelegt wird.

Durch die Auswahl der Primer für die Amplifikationsreaktion können neben den bisher identifizierten, bekannten Genomsequenzen von HIV, HCV und HBV auch ihre Subtypen erfaßt werden. Dies wird erfindungsgemäß dadurch gelöst, daß die verwendeten Primer aus einer hochkonservierten Genomregion des jeweiligen Virus ausgewählt werden. Für die Selektion des erfindungsgemäßen Ausgangsmaterials bzw. Zwischenprodukts mittels der LIF-PCR und der DTS-PCR kommen daher ausschließlich Primer zu Verwendung, deren Spezifität für alle relevanten Subtypen überprüft wird.

Im Rahmen einer epidemologischen Überwachung werden neu auftretende Virusstämme der Zielviren untersucht, ob sie die entsprechenden Templates, gegen die die verwendeten Primer gerichtet sind, enthalten. Aufgrund von ermittelten Veränderungen innerhalb einer bekannten Gruppe von Virusstämmen können dann entsprechend neue Primer für den quantitativen Nachweis von vorhandenen Kopien eines neuen Virusstammes synthetisiert werden. Dabei sollen die neusynthetisierten Primer eine mindestens gleich gute Empfindlichkeit des Test entsprechend der von schon erprobten Primern liefern.

Für neu auftretende Zielviren müssen entsprechende neue Primer für konservierte Nukleinsäuresequenzen entwickelt werden.

Zur Kontrolle des Verfahrens zum Nachweis bzw. zur Bestimmung von Nukleinsäuren in einer Probe werden vor oder während der Durchführung des Verfahrens der Probe ein oder mehrere interne Standards bzw. Referenzpräparationen zugesetzt, wobei die Standards zugleich mit gegebenenfalls vorhandenen viralen Genomen oder Genomsequenzen in ein und demselben Testgefäß bestimmt bzw. nachgewiesen werden.

Diese Vorgangsweise bietet die Möglichkeit einer noch exakteren Quantifizierung des Gehaltes an viralen Nukleinsäuren bzw. eine noch bessere Abschätzung der Nachweisgrenze des Amplifikationsverfahrens, insbesondere wenn der oder die internen Standards in einer Menge eingesetzt werden, die nahe der Nachweisgrenze der jeweiligen Amplifikationsreaktion liegt.

Die erfindungsgemäß zu einem qualitätsgesicherten Plasmapool vereinigten Einzelspenden können mit weiteren gleichartig qualitätsgesicherten Plasmapools vereinigt werden, so daß ein qualitätsgesicherter Minipool, Makropool oder Multimakropool zur Verfügung gestellt wird. Die Vorteile eines aus qualitätsgesicherten kleineren Pools hergestellten größeren Pools sind bereits ausreichend beschrieben worden.

Gemäß einein weiteren Aspekt betrifft die vorliegende Erfindung ein Verfahren gemäß den Ansprüchen zur Herstellung eines Plasmapools als qualitätsgesichertes Ausgangsmaterial mit einer gesicherten limitierten Belastung an vermehrungsfähigen Viren aus zwei oder mehr Einzelspenden, welches durch die folgenden Schritte gekennzeichnet ist:
- Entnehmen von Proben aus n Einzelspenden,
- Vereinigen der Einzelspenden-Proben zu m Probenpools und
- Detektieren der in diesen Probenpools vorhandenen Menge an viralen Genomen oder Genomsequenzen mittels eines quantifizierbaren, kontrollierten und nicht-inhibierten Verfahrens zum Nachweis bzw. zur Bestimmung von Nukleinsäuren,
wonach diejenigen Einzelspenden (n_{g}), von denen die detektierte Menge an viralen Genomen oder Genomsequenzen in dem Probenpool unter einem bestimmten Grenzwert liegt, zu einem gesicherten Plasmapool vereinigt werden und diejenigen Einzelspenden (nₐ), von denen die detektierte Menge an viralen Genomen oder Genomsequenzen in dem Probenpool größer oder gleich dem Grenzwert ist, einer Weiterbehandlung zugeführt oder ausgeschieden werden und wobei n und m positive ganze Zahlen sind. Eine Weiterbehandlung der Einzelspenden kann beispielsweise durch Abreicherung von viralen Genomäquivalenten oder durch Zumischen von Virus-neutralisierenden Antikörper-haltigen Fraktionen erfolgen.

In einem weiteren Verfahrensschritt können auch zur Weiterbehandlung der ausgeschiedenen nₐ Einzelspenden erneut Proben entnommen werden und diese Einzelspenden-Proben zu mₐ Probenpools vereinigt werden, wobei nₐ und mₐ positive ganze Zahlen sind, mₐ ≥ 2 ist und das Verhältnis mₐ:nₐ größer ist als das Verhältnis m:n, und in diesen Probenpools erneut die Menge an viralen Genomen oder Genomsequenzen mittels eines quantifizierbaren, kontrollierten und nicht-inhibierten Verfahrens zum Nachweis bzw. zur Bestimmung von Nukleinsäuren detektiert werden, wonach diejenigen Einzelspenden, von denen die detektierte Menge an viralen Genomen oder Genomsequenzen in dem Probenpool unter einem bestimmten Grenzwert liegt, zu einem gesicherten Plasmapool vereinigt werden und diejenigen Einzelspenden, von denen die detektierte Menge an viralen Genomen oder Genomsequenzen größer oder gleich dem Grenzwert ist, einer Weiterbehandlung zugeführt oder ausgeschieden werden. Dieses Verfahren kann solange wiederholt werden, bis die Anzahl der Einzelspenden, welche weiterzubehandeln oder auszuscheiden sind, eine festgelegte Zahl erreicht oder unterschritten hat.

Damit wird ein einfaches Verfahren zur Verfügung gestellt, welches es erlaubt, aus einem nahezu beliebig großen Plasmapool mit wenigen Tests etwa vorhandene Einzelplasmaspenden mit unzulässig hoher Belastung an vermehrungsfähigen Viren auszuscheiden. Auf der anderen Seite werden geeignete Einzelspenden, welche mit bisherigen Verfahren zusammen mit einer virusbelasteten Einzelspende ausgeschieden worden sind, wenn sie gemeinsam analysiert wurden, mit einfachen Verfahrensschritten erkannt und können zu einem fertigen Plasmaderivat verarbeitet werden.

Damit kann ein weiteres Ziel der Erfindung erreicht werden, nämlich auch aus einem großen Plasmapool einen einzigen verseuchten Spender auf einfache und kostensparende Weise herauszufinden, um ihn von weiteren Spenden auszuschließen und ihn sofort ärztlicher Hilfe zuzuleiten. Damit wird die Sicherheit der Spenderkolonie vergrößert.

Die Zahl der Einzelspenden, welche der dem erfindungsgemäßen Verfahren unterzogene Plasmapool umfaßt, beträgt in der Regel 2 bis 200.000 (n = 2 bis 200.000) und vorzugsweise 20 bis 20.000, besonders bevorzugt 200 bis 2000, insbesondere 200. Die Zahl m liegt dabei vor allem zwischen 1 und 100.000, vorzugsweise zwischen 2 und 1000. Die festgelegte Zahl an weiterzubehandelnden oder auszuscheidenden Einzelspenden beträgt praktischerweise 100, vorzugsweise 10, besonders bevorzugt 1. Im letzten Fall wird also die viruskontaminierte Einzelspende als solche identifiziert. In der Praxis ist dies jedoch nur aus Gründen der Identifizierung des Plasmaspenders angezeigt, es wird daher die festgelegte Zahl üblicherweise zwischen 10 und 100 liegen.

Die Probenpools können, falls dies erforderlich erscheint, zusätzlich zum Nachweis bzw. zur Bestimmung von Nukleinsäuren auf virusneutralisierende Antikörper gegen die zu untersuchenden Viren getestet werden.

Dabei werden in der Regel bei denjenigen Probenpools, in denen Antikörper gegen Viren gefunden wurden, die entsprechenden Einzelspenden vereinigt und weiter verwendet. Diejenigen Einzelspenden, von denen keine Antikörper im Probenpool festgestellt worden sind, werden dann einer Weiterbehandlung zugeführt oder ausgeschieden.

Bei einem bevorzugten Verfahren zur Herstellung von Arzneimitteln aus menschlichem Plasma wird mindestens noch von einem Zwischenprodukt im Laufe des Herstellungsverfahrens ein Qualitätssicherungsschritt angewendet.

Gemäß einer bevorzugten Ausführungsform wird der Qualitätssicherungsschritt im Zwischenprodukt, welches einem wesentlichen Virusabreicherungs- bzw. Virusinaktivierungsschritt unterzogen wird, durchgeführt. Am meisten bevorzugt ist die Qualitätssicherung von jedem Zwischenprodukt, welches einem wesentlichen Virusabreicherungs- bzw. Virusinaktivierungsschritt unterzogen werden soll.

Der Gegenstand der Erfindung ist in den Ansprüchen beschrieben.

Die Erfindung wird durch die nachfolgenden Beispiele, auf die sie jedoch nicht beschränkt sein soll, erläutert.

### Beispiele:

### 1. Allgemeines Prinzip der PCR

### 1.1. Allgemeines Prinzip der DTS-PCR (Dual-Targeting-Southern-Blot)

Nukleinsäuren werden mittels eines ersten spezifischen Primer-Paares mit PCR amplifiziert, die PCR-Produkte anschließend auf einem Agarosegel elektrophoretisch aufgetrennt und auf einen Filter geblottet. Die filtergebundenen PCR-Produkte werden mit einer Digoxigenin (DIG)-markierten Sonde, die innerhalb der amplifizierten Genomsequenz bindet, hybridisiert und nach einer Entwicklungsreaktion nachgewiesen. Die Bandenintensität wird quantitativ densitometrisch bestimmt. In einer weiteren PCR wird unter Verwendung eines zweiten, vom ersten Primer verschiedenen, Primer-Paares die Nukleinsäure amplifiziert und ebenfalls über Southern-Blot-Analyse detektiert. Durch die PCR zweier verschiedener Regionen einer Genomsequenz und deren Sichtbarmachung mittels Hybridisierung können falsch negative Ergebnisse reduziert und positive Resultate verifiziert werden.

### 1.2. Allgemeines Prinzip der LIF-PCR (Laser-induzierten Fluoreszenz-markierten)

Nukleinsäuren unterschiedlicher Herkunft wurden mittels PCR unter Verwendung von Primern, welche fluoreszierende Gruppen haben, amplifiziert. Die Analyse und Quantifizierung der erhaltenen amplifizierten Produkte wurden mit Hilfe eines automatischen DNA-Sequenzierers mit laserinduzierter Fluoreszenz-Meßeinrichtung (DNA-Sequenzierer 373A mit Gene Scan®-Software von Applied Biosystems) ausgeführt. Dieses Instrument ist in der Lage, die Fluoreszenzmarkierten PCR-Produkte mittels einer Gelelektrophorese in einem Polyacrylamidgel unter denaturierenden Bedingungen der Größe nach aufzutrennen, und deren Menge quantitativ zu bestimmen. Die Kopienzahl bestimmter Sequenzen in der Probe wird auf Grundlage der erhaltenen Intensitäten der PCR-Produkte von zu quantifizierender Nukleinsäure und mindestens zwei internen Standards bestimmt.

### 1.2.1. Extraktion der DNA viraler Partikel

500 µl der Probe werden für 20 min bei 70000 rpm in einer Ultrazentrifuge zentrifugiert. Das Pellet wird in 500 µl 10 mM Tris/HCl pH 8,0 und 10 µl Proteinase K (Boehringer Mannheim, 20 mg/ml), sowie 10 µl 20% SDS aufgelöst. Nach Inkubation über Nacht bei 37°C oder für 4 h bei 56°C wird eine bestimmte Menge an Standard-Nukleinsäure zugesetzt, die Probe nacheinander mit Phenol und Chloroform extrahiert und 10 µl Glykogen (Boehringer Mannheim, 20 mg/ml) zugesetzt. Anschließend wird mit Ethanol präzipitiert, zentrifugiert, das Pellet gewaschen und schließlich in Wasser wieder gelöst.

### 1.2.2. Extraktion viraler Rest-DNA

500 µl der Probe werden in 5 µl 10mM Tris/HCl pH 8,0 und 10 µl Proteinase K (20 mg/ml) aufgelöst. Nach Inkubation über Nacht bei 37°C oder für 4 h bei 56°C wird eine bestimmte Menge an Standard-Nukleinsäure zugesetzt, die Probe nacheinander mit Phenol und Chloroform extrahiert und 10 µl Glykogen (20 mg/ml) zugesetzt. Anschließend wird mit Ethanol präzipitiert, zentrifugiert, das Pellet gewaschen und schließlich in Wasser wieder gelöst.

### 1.2.3. Extraktion der RNA für die PCR

1 ml Plasma bzw. mit PBS verdünntes Plasma wird bei 70000 rpm 20 min zentrifugiert. Der Überstand wurde durch Absaugen entfernt. Das Pellet wurde in 1 ml Guanidiumisothiocyanat-Lösung (RNAzol^{?} der Firma Biotex) aufgenommen und 5 µl 1 mg/ml t-RNA aus Hefe und eine vorbestimmte Menge, z.B. 20 µl, Standard-RNA zugegeben. Es werden eine vorbestimmte Anzahl, z.B. 400 und 1200 Kopien, des Minus- und des Plus-RNA-Standards zugegeben und gevortext. Die Lösung wird 10 min bei 70°C erhitzt, dann 1/10 Volumen Chloroform zugegeben und für 10 min auf Eis inkubiert. Dann wird für 5 min in einer Tischzentrifuge zentrifugiert und der Überstand in neue Röhrchen transferiert. 500 µl Isopropanol werden zugegeben und 15 min auf -80°C gestellt. Anschließend wird 10 min zentrifugiert, 2x mit 70% Ethanol gewaschen und das Pellet in 50 µl Wasser aufgenommen. Für die RT-PCR wurden 5 µl eingesetzt.

### 1.2.4. PCR für den Nachweis von DNA

Der PCR-Ansatz enthält in bekannter Weise ein Aliquot der extrahierten Nukleinsäure, PCR-Puffer (Boehringer Mannheim), MgCl₂, dNTPs, Primer, Taq-Polymerase (Boehringer Mannheim, 5,0 E/µl) und Wasser. Die PCR wird gemäß den Angaben des Herstellers von Puffer und Enzym bzw. gemäß üblicher Arbeitsvorschriften (Mullis et al. Methods in Enzymology 155: 335, 1987) durchgeführt.

### 1.2.5. RT-PCR für den Nachweis von HIV-RNA

Der RT-PCR Ansatz enthält in bekannter Weise ein Aliquot der extrahierten Nukleinsäure in RT-Puffer von Perkin-Elmer, MgCl₂, dNTPs, den RT-Primer und rT.th.-Polymerase (Perkin-Elmer, 2,5 E/µl) und Wasser. Die RT wird gemäß den Angaben des Herstellers von Puffer und Enzym bzw. gemäß üblicher Arbeitsvorschriften (Mullis et al. Methods in Enzymology 155: 335, 1987) in einer PCR-Apparatur (GeneAmp PCR System 9600 der Firma Perkin-Elmer) durchgeführt.
Für die PCR werden noch MgCl₂, ein Chelatpuffer und der zweite Primer zugegeben. Dann wird die PCR nach den oben beschriebenen Angaben durchgeführt.

### 1.2.6. Analyse der Produkte

Für die Bestimmung und Quantifizierung der PCR-Produkte werden der PCR-Lösung 0,5 bis 1,0 µl entnommen und in einem 373 A Instrument der Firma Applied Biosystems gemäß den Angaben des Herstellers analysiert.

### 1.3. Southern blotting und Detektion der DTS-PCR-Produkte

Agarosegele wurden für die gelelektrophoretische Auftrennung der PCR-Produkte mit einer für das jeweilige Produkt bestimmten Konzentration hergestellt. Der Gellauf erfolgte in 1x Elektrophoreselaufpuffer. Die PCR-Produktproben wurden mit Ficoll/Bromphenolblau in 0,5 x Laufpuffer versetzt und die vorbereiteten Geltaschen beladen. Nach dem Gellauf wurden die PCR-Produkte durch Inkubation des Gels in 0,5 M NaOH/1,5M NaCl 1 h denaturiert und anschließend neutralisiert. Die DNA wurde auf Filter (Duralon™, Stratagene, La Jolla, Californien) transferiert und die Nukleinsäure wurde mittels UV-cross linking an die Membran gebunden. Die Membran wurde 1 h bei 68°C in Prähybridisierungspuffer (6 x SSC, 0,5% SDS, 5 x Denhardts, 100 µg/ml denaturierte DNA) inkubiert. Die Hybridisierung erfolgte mit einer Dioxigenin-markierten DNA-Sonde und anschließender Sichtbarmachung der Banden durch Immunfärbung mittels alkalische Phosphatase-konjugierter Antikörper, Nitro-Blue-Tetrazolium (NBT) und 5-Bromo-1-chlor-3-indolphosphat (BCIP). Die Auswertung der Blots erfolgte densitometrisch.

### 1.4. Verwendete Primer

Für die LIF-PCR verwendeten Primer sind in Tabelle 1.1. und Tab. 1.2. zusammengefaßt.
Die in der DTS-PCR eingesetzten Primer sind für HIV : SK38, SK 39, SK145 und SK431 (Ratner et al. 1985), für HCV: 32, R3, CHAA, R1, ConA1, ConaA2 und ConA (erhältlich von Chiron) und für HBV: HBV1a und HBV1b (Kaneko et al. 1989), sowie HBV4a und HBV4b (Carman et al. 1989).

### Tabelle 1: Verwendete Primer und Standardplasmide für die LIF-PCR und DTS-PCR Tabelle 1

**Tab. 1.1. Primer für die Amplifikation und Detektion in der LIF-PCR**

| **Oligonukleotid-Bezeichnung Orientierung** | **Sequenz (5'-3')** | **Virus** | **Position** |
|---|---|---|---|
| SK38 (+) | ATAATCCACCTATCCCAGTAGGAGAAAT | HIV-1 | 1551-1578^{b} |
| SK39 (-) | TTTGGTCCTTGTCTTATGTCCAGAATGC | HIV-1 | 1665-1638^{b} |
| HCV32 | CTGTGAGGAACTACTGTCTT | HCV | 45-64^{c} |
| HCVPT4 | CGGTTCCGCAGACCACCTATG | HCV | 158-139^{c} |
| +1780B | CATTGATCCTTATAAAGAATTTGGAGC | HBV | 1780-1806^{d} |
| -1950B | CCAGCAGAGAATTGCTTGCCTGAG | HBV | 1973-1950 |

| | | | |
|---|---|---|---|
| ^{b} Numerierung nach Ratner et al. (Nature 313:277-284, 1985) | | | |
| ^{c} Numerierung nach Han et al. (PNAS 88: 1711-1715, 1991) | | | |
| ^{d} Numerierung nach Fujiyama et al. (Nucleic Acids Res. 11: 4601-4610, 1983) | | | |

**Tab. 1.2.**

| Standardplasmide für die LIF-PCR | | |
|---|---|---|
| **Bezeichnung** | **Deletion/Insertion** | **Virus** |
| pgag1 | | HIV-1 |
| pgag -15 | del. 1593-1607^{b} | HIV-1 |
| pgag +12 | ins: +12bp Pos.1593^{b} | HIV-1 |
| pHCV-wt | | HCV |
| pHCV-7bp | del. 126-135^{c} | HCV |
| pHCV +8bp | ins. + 8bp Pos. 126^{c} | HCV |
| pHBV-wt | | HBV |
| pHBV-9bp | del. 1868-1876^{d} | HBV |
| pHBV+12bp | ins. +12 Pos. 1868^{d} | HBV |

| | | |
|---|---|---|
| ^{b} Numerierung nach Ratner et al. (Nature 313:277-284, 1985) | | |
| ^{c} Numerierung nach Han et al. (PNAS 88: 1711-1715, 1991) | | |
| ^{d} Numerierung nach Fujiyama et al. (Nucleic Adds Res. 11: 4601-4610, 1983) | | |

### Beispiel 2:

### 2.1. Quantifizierung von HIV RNA in Plasmaproben von Spendern durch LIF-PCR und DTS-PCR

Von HIV-seronegativen, gesunden Probanden und HIV-seronegativen, p24-Antigen-positiven primär-infizierten Probanden wurde eine Plasmaspende genommen. Plasmaproben der Spender wurden mittels LIF-PCR und DTS-PCR getestet. Die Probentestung mittels DTS-PCR erfolgte mit den Primerpaaren SK145/431 (Primerpaar 1) und SK38/39 (Primerpaar 2) (Tab.1.1); Proben mit positiven Hybridisierungssignalen wurden mit der LIF-PCR weitergetestet. Für die Quantifizierung mittels LIF-PCR wurden die Primer SK38 und SK39 (Tab. 1.1.) verwendet, welche in den cDNA-Sequenzen des HIV-1 binden und durch RT-PCR von der Wildtyp-RNA ein 115 bp großes PCR-Produkt ergeben. Als Standardplasmide wurden pgag1, pgag -15 und pgag +12 (Tab. 1.2.) eingesetzt, die RT-PCR-Produkte der Länge von 115 bp (pgag1), 100 bp (pgag-15) und 127 bp (pgag+12) ergeben. Die Standardplasmide wurden mit der Probe koamplifiziert und auf dem Gene Scan® koanalysiert und die Kopienzahl/ml ermittelt. Die Resultate der quantitativen Auswertung sind in Tabelle 2 zusammengefaßt.

**Tabelle 2**

| Probanden | LIF-PCR Kopien/ml | DTS-PCR PCR/Primerpaar 1 Hybridisierungssignal | PCR/Primerpaar 2 Hybridisierungssignal |
|---|---|---|---|
| HIV-sero-/p24 positiv #1 | 3,4 x 10⁵ | + | + |
| HIV-sero-/p24 positiv #2 | 2,8 x 10⁵ | + | + |
| HIV-sero-/p24 positiv #3 | 2,7 x 10⁶ | + | + |
| HIV-sero-/p24 positiv #4 | 1,7 x 10⁷ | + | + |
| HIV-sero-/p24 positiv #5 | 5,5 x 10⁵ | + | + |
| HIV-sero-/p24 positiv #6 | 8,6 x 10⁶ | + | + |
| HIV-sero-/p24 positiv #7 | 2,3 x 10⁷ | + | + |
| HIV-sero-/p24 positiv #8 | 4,7 x 10⁵ | + | + |

Die minimale Belastung bei HIV-seronegativen, p24 Antigen positiven Plasmaspendern wurde mit 2,8 x 10⁵ und die maximale mit 2,3 x 10⁷ Kopien/ml ermittelt.

### 2.2. Quantifizierung von HCV RNA in Plasmaproben von Spendern durch LIF-PCR und DTS-PCR

Von HCV-seronegativen, gesunden Probanden und HCV-primär-infizierten Probanden wurde eine Plasmaspende genommen. Plasmaproben der Spender wurden mittels LIF-PCR und DTS-PCR getestet. Die Probentestung mittels DTS-PCR erfolgte mit den Primerpaaren 32/R3 (Primerpaar 1) und CHAA/R1 (Primerpaar 2) (Chiron); Proben mit positiven Hybridisierungssignalen wurden mit der LIF-PCR weitergetestet. Für die Quantifizierung mittels LIF-PCR wurden die Primer HCV32 und HCVPT4 (Tab. 1.1.) verwendet, welche in den cDNA-Sequenzen des HCV binden und durch RT-PCR von der Wildtyp-RNA ein 114 bp großes Produkt ergeben. Als Standardplasmide wurden pHCVwt, pHCV-7 und pHCV+8 (Tab. 1.2.) eingesetzt, die RT-PCR-Produkte der Länge von 114 bp (pHCVwt), 107 bp (pHCV-7) und 122 bp (pHCV+8) ergeben. Die Standardplasmide wurden mit der Probe koamplifiziert und auf dem Gene Scan® koanalysiert und die Kopienzahl/ml ermittelt. Die Resultate der quantitativen Auswertung sind in Tabelle 3 zusammengefaßt.

**Tabelle 3**

| Probanden | LIF-PCR Kopien/ml | DTS-PCR PCR/primerpaar 1 Hybridisierungssignal | PCR/Primerpaar 2 Hybridisierungssignal |
|---|---|---|---|
| HCV-sero-/# 1 | 1,3 x 10⁵ | + | + |
| HCV-sero-/# 2 | 3,8 x 10⁵ | + | + |
| HCV-sero-/# 3 | 1,6 x 10⁶ | + | + |
| HCV-sero-/# 4 | 2,2 x 10⁴ | + | + |
| HCV-sero-/# 5 | 8,2 x 10⁴ | + | + |
| HCV-sero-/# 6 | 6,2 x 10⁵ | + | + |
| HCV-sero-/# 7 | 9,4 x 10⁴ | + | + |
| HCV-sero+/# 8 | 2,3 x10² | + | + |

Die minimale Belastung bei HCV-seronegativen, primärinfizierten Plasmaspendern wurde mit 2,2 x 10⁴ Kopien/ml und die maximale mit 1,6 x 10⁶ Kopien/ml ermittelt.

### 2.3. Quantifizierung von HBV DNA in Plasmaproben von Spendern durch LIF-PCR und DTS-PCR

Von HBV-seronegativen, gesunden Probanden und primär-infizierten, HBsAg-positiven, anti-HBsAg-seronegativen und HBsAg-positiven, anti-HBsAg-seropositiven Probanden wurde eine Plasmaspende genommen. Plasmaproben der Spender wurden mittels LIF-PCR und DTS-PCR getestet. Die Probentestung mittels DTS-PCR erfolgte mit den Primerpaaren HBV1a/HBV1b (Primerpaar 1) und HBV4a/HBV4b (Primerpaar 2) (Carman et al. 1989); Proben mit positiven Hybridisierungssignalen wurden mit der LIF-PCR weitergetestet. Für die Quantifizierung mittels LIF-PCR wurden die Primer HBV+1780B und HBV-1950B (Tab. 1.1.) verwendet, welche in den cDNA-Sequenzen des HBV-Genoms binden und durch RT-PCR von der Wildtyp-RNA ein 182 bp großes PCR-Produkt ergeben. Als Standardplasmide wurden pHBVwt, pHBV-9 und pHBV+12 (Tab. 1.2.) eingesetzt, die RT-PCR-Produkte der Länge von 182 bp (pHBVwt), 173 bp (pHBV-9) und 194 bp (pHCV+12) ergeben. Die Standardplasmide wurden mit der Probe koamplifiziert und auf dem Gene Scan® koanalysiert und die Kopienzahl/ml ermittelt. Die Resultate der quantitativen Auswertung sind in Tabelle 4 zusammengefaßt.

**Tabelle 4**

| Probanden | LIF-PCR Kopien/ml | DTS-PCR PCR/Primerpaar 1 Hybridisierungssignal | PCR/Primerpaar 2 Hybridisierungssignal |
|---|---|---|---|
| HBV-sero-/HBsAg positiv #1 | 1,4 x 10⁵ | + | + |
| HBV-sero-/HBsAg positiv #2 | 2,8 x 10⁵ | + | + |
| HBV-sero-/HBsAg positiv #3 | 3,5 x 10⁵ | + | + |
| HBV-sero-/HBsAg positiv #4 | 1,3 x 10⁵ | + | + |
| HBV-sero-/HBsAg positiv #5 | 1,9 x 10⁵ | + | + |
| HBV-sero-/HBsAg positiv #6 | 2,3 x 10⁶ | + | + |
| HBV-sero+/HBsAg positiv #7 | 5,6 x 10⁴ | + | + |

Die minimale Belastung bei HBV-seronegativen primärinfizierten Plasmaspendern wurde mit 1,4 x 10⁵ Kopien/ml und die maximale mit 1,3 x 10⁸ Kopien/ml ermittelt.

### Beispiel 3:

### 3.1. Testen von Plasmapools verschiedener Größe auf HIV-1 RNA

Plasmaproben von 10, 50, 100, 500, 1000 und 2000 HIV-seronegativen, p24 Antigen-negativen, gesunden Einzelspendern wurden zu einem Probenpool gemischt. Die einzelnen Poolgrößen wurden mit je einer Plasmaprobe eines HIV-Primärinfizierten mit einer nach Beispiel 1 ermittelten hohen Belastung von 2,3 x 10⁷ Kopien/ml (Probe 1), eines HIV-Primärinfizierten mit einer nach Beispiel 1 ermittelten minimalen Belastung von 2,8 x 10⁵ Kopien/ml (Probe 2) und als Kontrolle mit einer definierten Präparation von HIV RNA mit 1 x 10⁴ Kopien/ml (Probe 3) und 1 x 10³ Kopien/ml (Probe 4) gemischt. Für die jeweilige Poolgröße wurde mittels LIF-PCR die quantifizierbare virale Kopienzahl/ml ermittelt. Die Ergebnisse der PCR-Auswertung sind in Tabelle 5.1. zusammengefaßt.

### Ergebnis:

Tabelle 5.1. zeigt, daß in einem Plasmapool aus 10, 50, 100 und 500 Einzelspenden sowohl eine virale Kontamination mit einer primärinfizierten anti-HIV-seronegativen, p24-Antigen-positiven Einzelspende mit einer hohen (2,3 x 10⁷ Kopien/ml) als auch mit einer minimalen (3,8 x 10⁵ Kopien/ml) Belastung mittels PCR nachgewiesen werden kann. Bei einem Probenpool von 1000 und 2000 Einzelspendern konnte eine Kontamination, verursacht durch eine hoch HIV-RNA belastete Spende, ebenfalls detektiert werden; hingegen konnte eine Kontamination mit einer minimal belasteten Spende in diesen Poolgrößen nicht mehr detektiert werden. Bei einer HIV-RNA Belastung, verursacht durch eine Kontamination mit 1 x 10⁴ Kopien/ml in der Einzelspende war ein Nachweis bis zur einer Pool-Größe von 10 Einzelspenden möglich. Eine Detektion von viralen Genomsequenzen bei einer Kontamination von 1 x 10³ Kopien/ml in der Einzelspende war in keiner der getesteten Poolgrößen möglich.

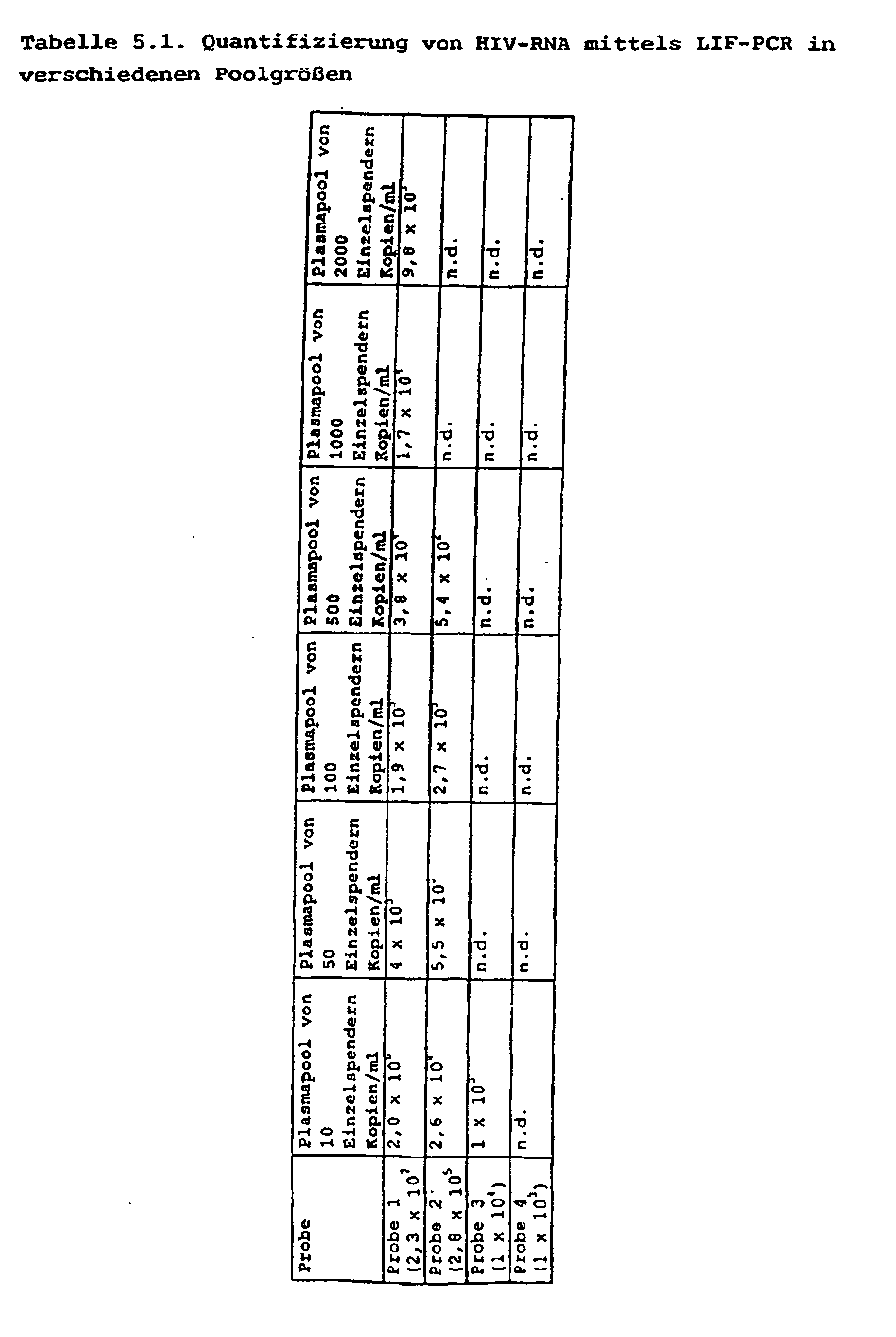

### 3.2. Erhöhung der Sensitivität durch Probenkonzentrierung

Um die Sensitivität in den Poolgrößen, bei denen keine HIV-RNA-Kopien nachgewiesen werden konnten zu steigern, wurden für die PCR-Reaktion 10fach konzentrierte Proben der gepoolten Plasmen eingesetzt. Dazu wurde jeweils das 10fache Volumen des in Beispiel 3.1. eingesetzten Probenvolumens der Proben 2, 3 und 4 der einzelnen Plasmapools durch Zentrifugation konzentriert, in Puffer mit 1/10 des Ausgangsvolumens resuspendiert und für die PCR-Reaktion eingesetzt. Die Ergebnisse der PCR-Auswertung sind in Tabelle 5.2. zusammengefaßt.

Durch die 10fache Konzentrierung des Ausgangsprobenvolumens für die PCR konnte die Minimal-Belastung eines Pools von 2000 Einzelspendern mit einer primär-HIV infizierten Spende (2,8 x 10⁵ Kopien/ml) detektiert werden. Ebenfalls konnte die Sensitivität des Nachweises bei Plasmapools von 50 und 100 Einzelspendern mit einer viralen Kontamination von 1 x 10⁴ Kopien/ml in einer Einzelspende gesteigert werden. Eine virale Kontamination mit 1 x 10³ Kopien/ml in einer Einzelspende konnte lediglich in der kleinsten Poolgröße von 10 Einzelspendern nachgewiesen werden.

In einem weiteren Versuch wurde, um den Nachweis von viralen Genomen in der nächsthöheren Poolgröße zu verbessern, das 100fache des in Beispiel 3.1. verwendeten Ausgangsvolumens der Proben 3 und 4 konzentriert, in Puffer mit 1/100 des Ausgangsvolumens resuspendiert und für die PCR eingesetzt. Die Ergebnisse der PCR-Auswertung sind in Tabelle 5.3. zusammengefaßt.

Durch die 100fache Aufkonzentrierung der Plasmaprobe konnte in einem Plasmapool der Größe 500, 1000 und 2000 Einzelspendern noch eine virale HIV-Genom-Belastung von 1 x 10⁴ Kopien/ml in einer Einzelspende nachgewiesen werden.

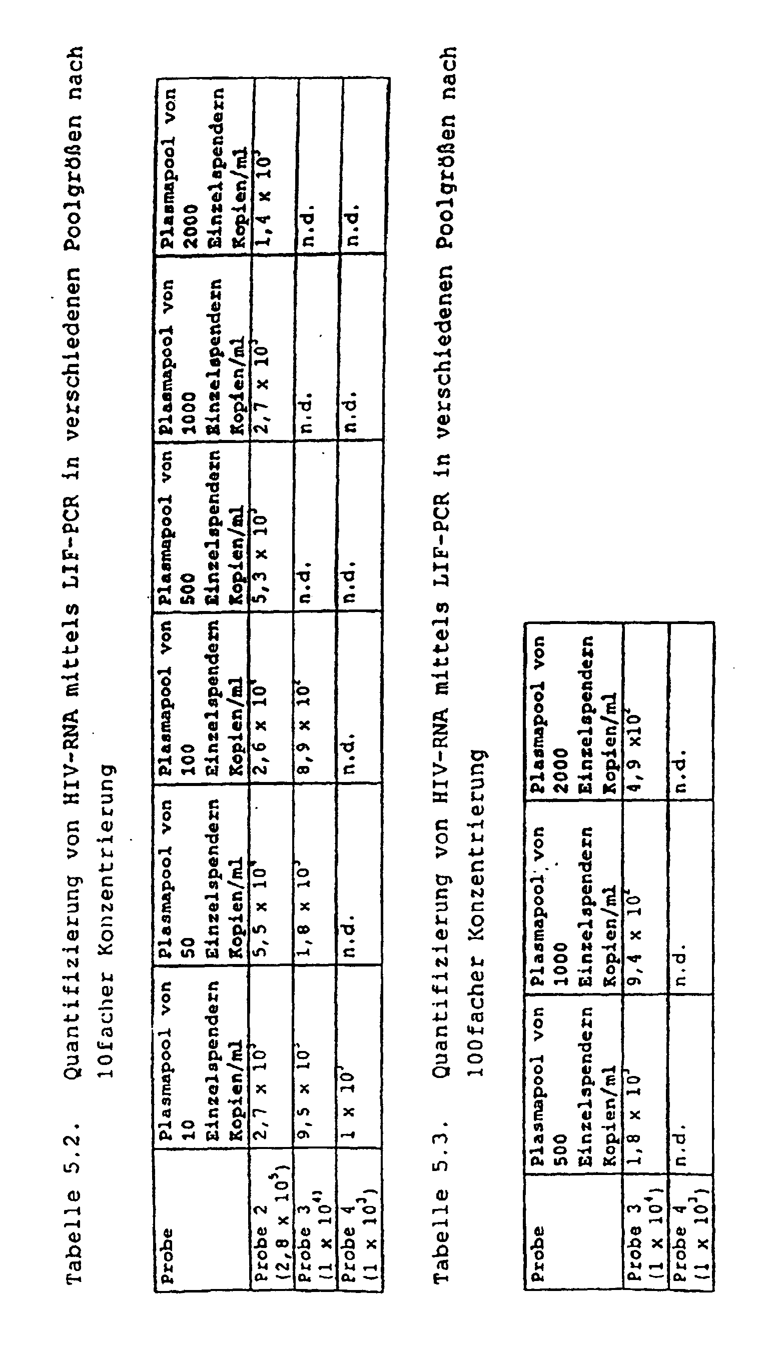

### Beispiel 4:

### 4.1. Testen von Plasmapools verschiedener Größe auf HCV RNA

Plasmaproben von 10, 50, 100, 500 und 1000HCV-seronegativen, gesunden Einzelspendern wurden zu einem Probenpool gemischt. Die einzelnen Poolgrößen wurden mit je einer Probe aus einer Plasmaspende eines MCV-Primärinfizierten mit einer nach Beispiel 1 ermittelten hohen Belastung von 1,6 x 10⁶ Kopien/ml (Probe 1), eines Primärinfizierten mit einer nach Beispiel 1 ermittelten minimalen Belastung von 2,2 x 10⁴ Kopien/ml (Probe 2) und als Kontrollen mit einer definierten Präparation von HCV mit 1 x 10³ Kopien/ml (Probe 3) und 5 x 10² Kopien/ml (Probe 4) gemischt. Mittels PCR wurde die in der jeweiligen Poolgröße quantifizierbare Kopienzahl/ml ermittelt. Die Ergebnisse der PCR-Auswertung sind in Tabelle 6.1. zusammengefaßt.

### Ergebnis:

Tabelle 6.1. zeigt, daß in einem Plasma-Pool bestehend aus 10, 50, 100, 500 und 1000 Einzelspenden eine virale Kontamination mit einer primärinfizierten Spende mit einer hohen Belastung von 1,6 x 10⁶ Kopien/ml nachgewiesen werden kann. Bei den Poolgrößen von 100, 500 und 1000 Einzelspendern konnte HCV-RNA, verursacht durch eine einzelne, minimal belastete Spende (2,2 x 10⁴ Kopien/ml), nicht mehr nachgewiesen werden. Eine Detektion von HCV-Genomäquivalenten ist bei einer Belastung von 2,2 x 10⁴ Kopien/ml in einer Einzelspende bis zu einer Poolgröße von 10 und 50 Spendern möglich. Viralen Genomäqui-valentbelastungen mit geringerer HCV-Kopienzahl von etwa 1 x 10³ bzw. 5 x 10² Kopien/ml in einer Einzelspende konnten in keiner der getesteten Poolgrößen detektiert werden.

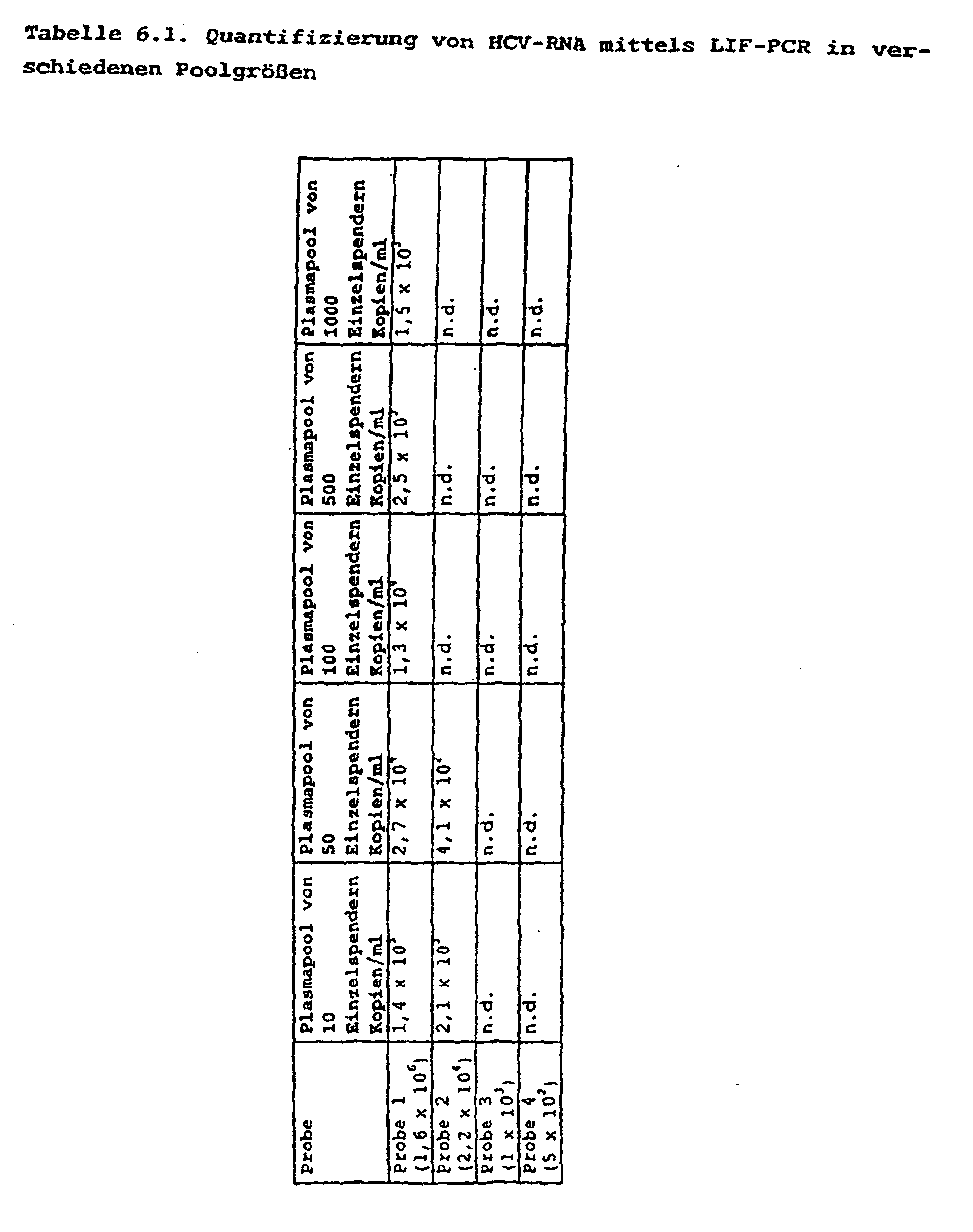

### 4.2. Erhöhung der Sensitivität durch Probenkonzentrierung

Um die Sensitivität in den Poolgrößen, bei denen keine HCV-RNA-Kopien nachgewiesen werden konnten zu steigern, wurden für die PCR-Reaktion 10fach konzentrierte Proben der gepoolten Plasmen eingesetzt. Dazu wurde jeweils das 10fache Volumen des in Beispiel 4.1. eingesetzten Probenvolumens der Proben 2, 3 und 4 der einzelnen Plasmapools durch Zentrifugation konzentriert, in Puffer mit 1/10 des Ausgangsvolumens resuspendiert und für die PCR-Reaktion eingesetzt. Die Ergebnisse der PCR-Auswertung sind in Tabelle 6.2. zusammengefaßt.

Durch die 10fache Konzentrierung des Ausgangsprobenvolumens für die PCR konnte die Minimai-Belastung eines Pools von 10, 50, und 500 Einzelspendern mit einer primär-HCV infizierten Spende (2,2 x 10⁴ Kopien/ml) detektiert werden. Ebenfalls konnte die Sensitivität des Nachweises einer viralen Kontamination mit 1 x 10³ bzw. 5 x 10² Kopien/ml in einer Einzelspende bei Plasmapools von 10 Einzelspendern erhöht werden.

In einem weiteren Versuch wurde, um den Nachweis von viralen Genomen in der nächsthöheren Poolgröße zu steigern, das 100fache des in Beispiel 4.1. verwendeten Ausgangsvolumens von Probe 3 und 4 konzentriert, in Puffer mit 1/100 des Ausgangsvolumens resuspendiert und für die PCR eingesetzt. Die Ergebnisse der PCR-Auswertung sind in Tabelle 6.3. zusammengefaßt.

Durch die 100fache Aufkonzentrierung der Plasmaprobe konnte in einem Plasmapool der Größe von 100 Einzelspendern noch eine virale HCV-Genom-Belastung mit 1 x 10³ und 5 x 10² Kopien/ml in einer Einzelspende nachgewiesen werden.

**Tabelle 6.2.**

| Quantifizierung von HCV-RNA mittels LIF-PCR in verschiedenen Poolgrößen nach 10facher Konzentrierung | | | | |
|---|---|---|---|---|
| Probe | Plasmapool von 10 Einzelspendern Kopien/ml | Plasmapool von 50 Einzelspendern Kopien/ml | Plasmapool von 100 Einzelspendern Kopien/ml | Plasmapool von 500 Einzelspendern Kopien/ml |
| Probe 2 (2,2 x10⁴) | 2,0 x 10⁴ | 4,1 x 10³ | 2,1 x 10³ | 4,0 x 10² |
| Probe 3 (1 x 10³) | 9,5 x 10² | n.d. | n.d. | n.d. |
| Probe 4 (5 x 10²) | 4,8 x10² | n.d. | n.d. | n.d. |

**Tabelle 6.3.**

| Quantifizierung von HCV-RNA mittels LIF-PCR in verschiedenen Poolgrößen nach 100facher Konzentrierung | | | | |
|---|---|---|---|---|
| Probe | Plasmapool von 100 Einzelspendern Kopien/ml | Plasmapool von 500 Einzelspendern Kopien/ml | Plasmapool von 1000 Einzeispendern Kopien/ul | Plasmapool von 2000 Einzelspendern Kopien/ml |
| Probe 3 (1 x 10³) | 9,5 x 10² | n.d. | n.d. | n.d. |
| Probe 4 (5 x 10²) | 4,7 x 10² | n.d. | n.d. | n.d. |

### Beispiel 5:

### 5.1. Testen von Plasmapools verschiedener Größe auf HBV DNA

Plasmaproben von 10, 50, 100, 500, 1000 und 2000 HBV-seronegativen, gesunden Einzelspendern wurden zu einem Probenpool gemischt. Der Pool wurde mit je einer Probe aus einer Plasmaspende eines HBV-Primärinfizierten mit einer nach Beispiel 1 ermittelten hohen Belastung von 1,3 x 10⁸ Kopien/ml (Probe 1), eines Primärinfizierten mit einer nach Beispiel 1 ermittelten minimalen Belastung von 1,4 x 10⁴ Kopien/ml (Probe 2) und als Kontrolle mit einer definierten Präparation von HBV mit 5 x 10³ Kopien/ml (Probe 3) und 1 x 10³ Kopien/ml (Probe 4) gemischt. Mittels PCR wurde die in der jeweiligen Poolgröße quantifizierbare Kopienzahl/ml bestimmt. Die Ergebnisse der PCR-Auswertung sind in Tabelle 7.1. zusammengefaßt.

Tabelle 7.1. zeigt, daß in einem Plasma-Probenpool aus 10, 50, 100, 500, 1000 und 2000 Einzelspenden eine virale Kontamination mit einer HBV-primärinfizierten Spende mit einer hohen Genom-Belastung nachgewiesen werden kann. Bei den Poolgrößen von 500, 1000 und 2000 Einzelspendern konnte HBV-DNA, verursacht durch eine einzelne, minimal belastete Spende (1,4 x 10⁴ Kopien/ml) nicht mehr nachgewiesen werden. Eine Detektion von HBV-Genomäquivalenten mit einer Belastung von 5 x 10³ Kopien/ml in einer Einzelspende war lediglich in den Poolgrößen von 10 und 50 Spendern und mit einer Belastung von 1 x 10³ Kopien/ml in einer Einzelspende bis zur einer Größe von 10 Spendern möglich.

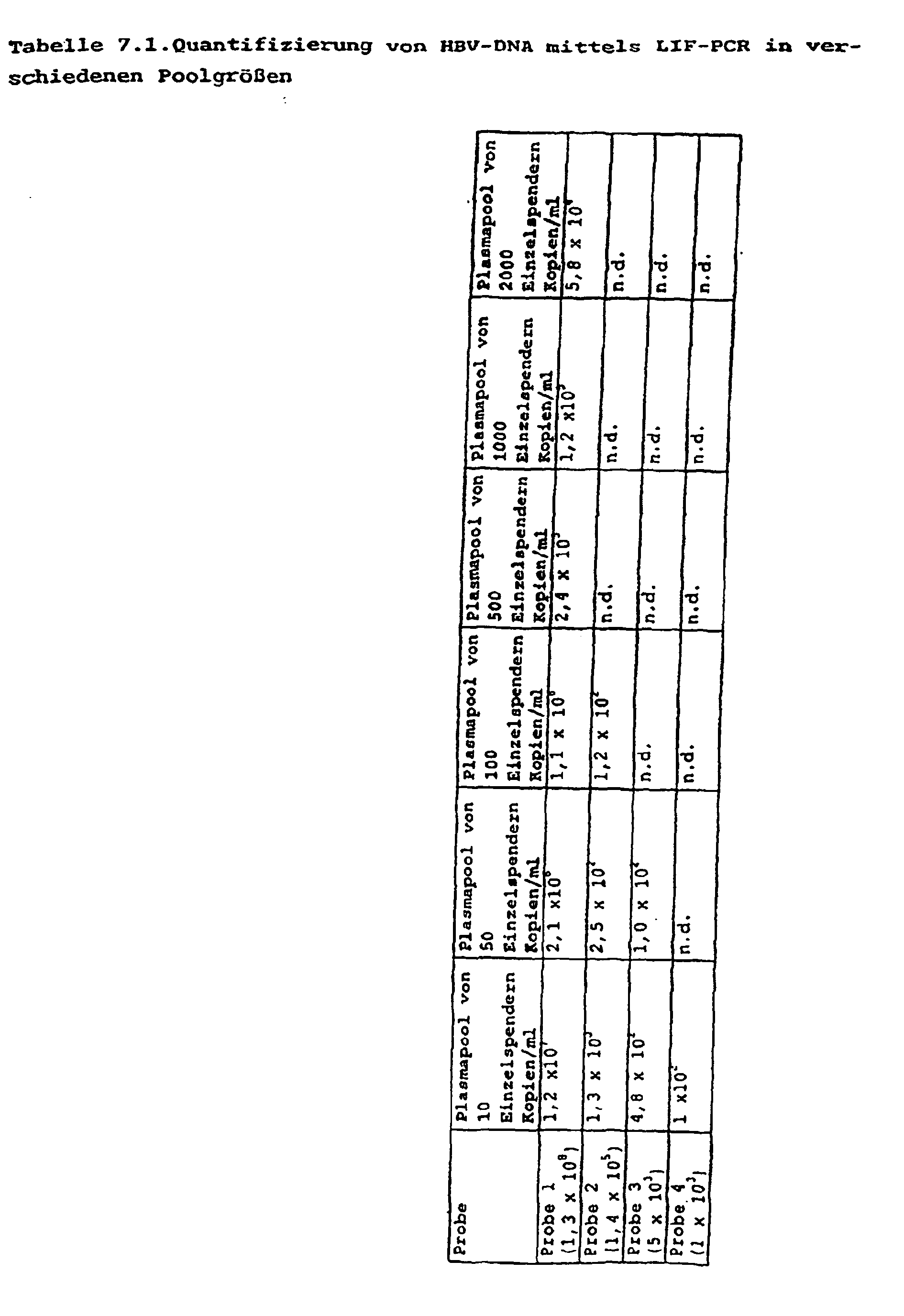

### 5.2. Erhöhung der Sensitivität durch Probenkonzentrierung

Um die Sensitivität in den Poolgrößen, bei denen keine HBV-DNA-Kopien nachgewiesen werden konnten zu steigern, wurden für die PCR-Reaktion 10fach konzentrierte Proben der gepoolten Plasmen eingesetzt. Dazu wurde jeweils das 10fache Volumen des in Beispiel 5.1. eingesetzten Probenvolumens der Probe 2, 3 und 4 der einzelnen Plasmapools durch Zentrifugation konzentriert, in Puffer mit 1/10 des Ausgangsvolumens resuspendiert und für die PCR-Reaktion eingesetzt. Die Ergebnisse der PCR-Auswertung sind in Tabelle 7.2. zusammengefaßt.

Durch die 10fache Konzentrierung des Ausgangsprobenvolumens für die PCR konnte die Minimal-Belastung eines Pools von 500 Einzelspendern mit einer primär-HBV infizierten Spende mit 1,4 x 10⁴ Kopien/ml detektiert werden. Ebenfalls konnte die Sensitivität des Nachweises einer viralen Kontamination einer Einzelspende von 5 x 10³ Kopien/ml bei Plasmapools von 100 und 500, und mit einer Kontamination einer Einspende von 1 x 10³ Kopien/ml bei einer Poolgröße von 50 und 100 Einzelspendern erhöht werden.

In einem weiteren Versuch wurde, um den Nachweis von viralen Genomen in der nächsthöheren Poolgröße zu steigern, das 100fache des in Beispiel 5.1. verwendeten Ausgangsvolumens von Probe 3 und 4 konzentriert, in Puffer mit 1/100 des Ausgangsvolumens resuspendiert und für die PCR eingesetzt (Tabelle 7.3.).

### Ergebnis:

Durch die 100fache Aufkonzentrierung der Plasmaprobe konnte in einem Plasmapool der Größe von 500 und 1000 Einzelspendern noch eine virale HIV-Genom-Belastung einer Einzelspende von 1 x 10³ Kopien/ml nachgewiesen werden.

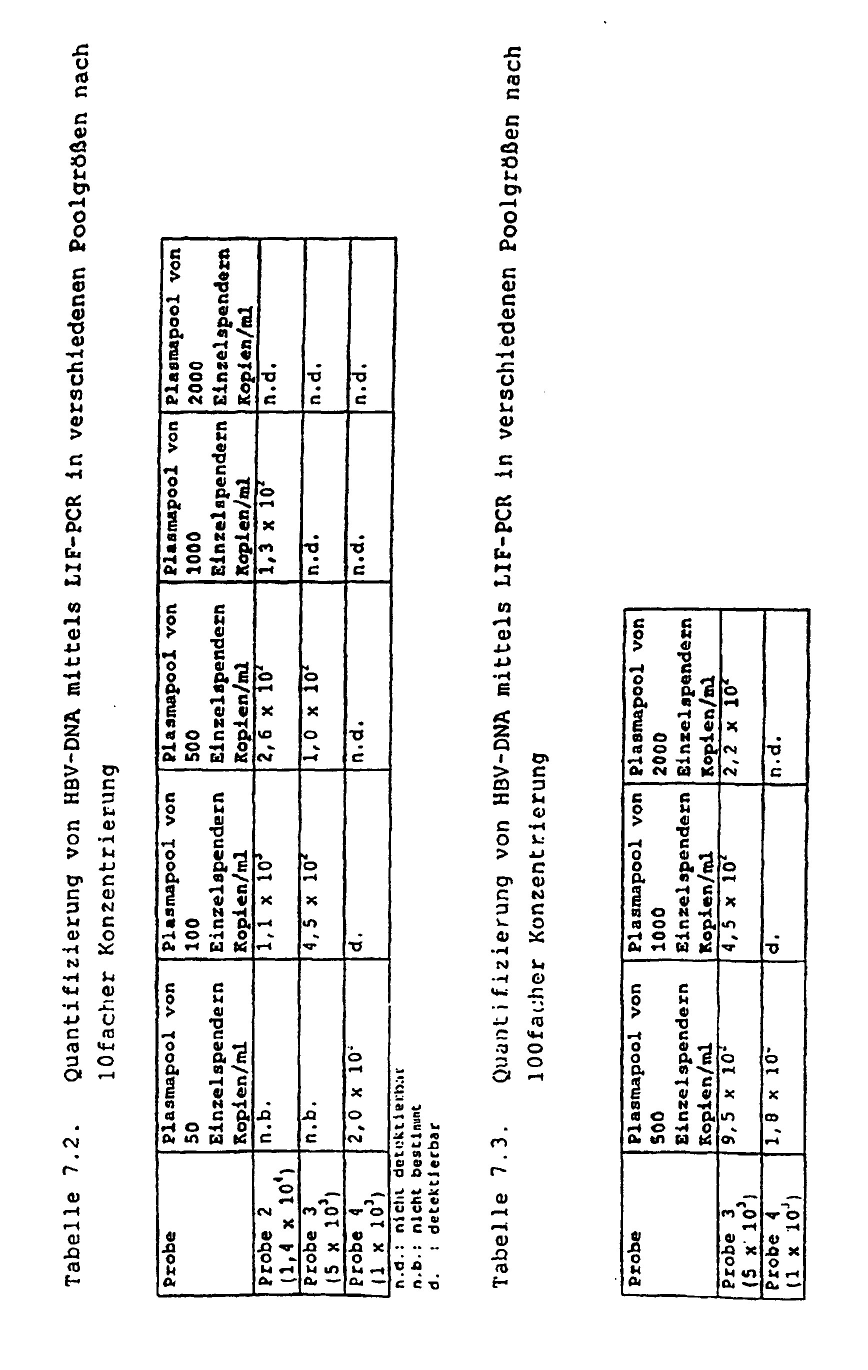

### Beispiel 6:

### Testen von Plasmapools verschiedener Größe auf HIV und HCV RNA

Plasmaproben von 10, 50, 100, 500, 1000 und 2000 HIV-und HCV seronegativen, gesunden Einzelspendern wurden zu einem Plasma-Probenpool gemischt. Die jeweilige Poolgröße wurde mit je einer Probe aus einer Plasmaspende eines HIV-Primärinfizierten und eines HCV-Primärinfizierten mit einer nach Beispiel 2.1 und 2.2. ermittelten hohen Belastung von HIV mit 2,3 x 10⁷ Kopien/ml und von HCV mit 1,6 x 10⁶ Kopien/ml (Probe 1), mit einer nach Beispiel 2.1. und 2.2. ermittelten minimalen Belastung von HIV mit 2,8 x 10⁵ Kopien/ml und von HCV mit 2,2 x 10⁴ Kopien/ml (Probe 2) und als Kontrolle mit einer definierten Präparation von HIV mit 1 x 10⁴ Kopien/ml und HCV mit 1 x 10³ Kopien/ml (Probe 3) und einer Präparation von HIV mit 1 x 10³ Kopien/ml und HCV mit 2 x 10² Kopien/ml (Probe 4) gemischt. Die jeweilige Poolgrößen wurde mittels LIF-PCR unter Verwendung der für HIV bzw. HCV spezifischen Primern auf die Anwesenheit von HIV bzw. HCV spezifischer Nukleinsäure getestet. HIV- und HCV-spezifische Genomsequenzen konnten bei einer Kontamination mit einer Maximalbelastung von 2,3 x 10⁷ HIV-Kopien/ml und 1,6 x 10⁶ HCV Kopien/ml durch eine Einzelspende bis zu einer Poolgröße von 1000 Einzelspendern nachgewiesen werden. HIV-Genomäquivalente wurden bei einer Kontamination mit einer gering belasteten Spende noch in einer Poolgröße von 500 Einzelspenden gefunden werden, wogegen HCV-Genomaquivalente nur in einem Pool von 50 Einzelspendern gefunden werden konnte. Die Sensitivität für den Nachweis von HCV Genomsequenzen liegt damit in den getesten Pools um eine log-Stufe unterhalb der für HIV-Genomsequenzen.

### 7. Nachweis des neutralisierenden Effekts einer HAV-immunisierten Spende auf die HAV Virusbelastung in einem Pool

Das neutralisierende Potential einer Plasmaspende eines HAV-immunisierten Spenders kann durch in vitro Neutralisationstests gezeigt werden.
Zum Nachweis des neutralisierenden Effektes von HAV-Antikörpern auf im Plasma vorhandene Hepatitis A-Viren wurde ein Plasmapool von 100 präimmunisierten HAV-Spendern mit einer protektiven Immunität getestet und der Pool mit HAV (Titer von 10^{6.4} TCID₅₀/ml) gemischt. Nach einer 1stündigen Inkubation bei 37°C wurde der HAV-Titer bestimmt. Durch die im Plasmapool vorhandenen HAV-Antikörper konnte die HAV-Infektiösität um ≥ 4.6 log-Stufen reduziert werden. Dadurch wird gezeigt, daB durch im Plasma von immunisierten Spendern vorhandene protektive Antikörper die Virusbelastung in einem größeren Plasmapool neutralisiert werden kann.

## Patentansprüche

1. Verfahren zur Herstellung eines Plasmapools von etwa 2000 bis 200000 Einzelspenden zum Fraktionieren, **gekennzeichnet durch** die folgenden Verfahrensschritte:
(a) Prüfung von virusneutralisierenden Antikörpern gegen die zu untersuchenden Viren oder **durch** Prüfung einer protektiven Immunität beim Spender des Ausgangsmaterials und **durch**
(b)Bestimmung der Genomäquivalente an vermehrungsfähigen HIV und HCV, sowie gegebenfalls weiterer vermehrungsfähige haematogenen Viren, insbesondere HAV und HBV,**durch**
- Entnehmen von Proben aus n Einzelspenden,
- Vereinigen der Einzelspenden-Proben zu m Probenpools und
- Detektieren der in diesen Probenpools vorhandenen Menge an viralen Genomen oder Genomsequenzen mittels eines quantifizierbaren, kontrollierten und nicht-inhibierten PCR-(Polymerasekettenreaktions-)Verfahrens zum Nachweis bzw. zur Bestimmung von Nukleinsäuren, wobei die festgestellte Menge an Genomäquivalenten nur dann den wahren Wert der Belastung an vermehrungsfähigen Viren angibt, wenn alle Genomäquivalente von aktiven Viren stammen, und wobei gegebenenfalls vorhandene Inhibitoren der PCR-Reaktion von dem PCR-Verfahren entfernt oder abgereichert werden und wobei vor oder während der Durchführung des PCR-Verfahrens ein oder mehrere interne Standards bzw. Referenzpräparationen der Probe zugesetzt werden, wobei die Standards zugleich mit gegebenenfalls vorhandenen viralen Genomen oder Genomsequenzen in ein und demselben Testgefäß bestimmt bzw. nachgewiesen werden.
wonach diejenigen Einzelspenden (n_{g}), von denen die detektierte Menge an viralen Genomen oder Genomsequenzen in dem Probenpool unter einem bestimmten Grenzwert liegt, zu einem qualitätsgesicherten Plasmapool vereinigt werden und diejenigen Einzelspenden (nₐ), von denen die detektierte Menge an viralen Genomen oder Genomsequenzen in dem Probenpool größer oder gleich dem Grenzwert ist, einer Weiterbehandlung zugeführt oder ausgeschieden werden und wobei n und m positive ganze Zahlen sind.

2. Verfahren nach Anspruch 1, **dadurch gekennzeichnet, dass**
- zur Weiterbehandlung der ausgeschiedenen nₐ Einzelspenden erneut Proben entnommen werden und diese Einzelspenden-Proben zu mₐ Probenpools vereinigt werden, wobei nₐ und mₐ positive ganze Zahlen sind, mₐ ≥ 2 ist, und das Verhältns mₐ:nₐ größer ist als das Verhältnis m:n, und
- in diesen Probenpools erneut die Menge an viralen Genomen oder Genomsequenzen mittels eines quantifizierbaren, kontrollierten und nicht-inhibierten Verfahrens zum Nachweis bzw. Bestimmung von Nukleinsäuren detektiert wird,
wonach diejenigen Einzelspenden, von denen die detektierte Menge an viralen Genomen oder Genomsequenzen in dem Probenpool unter einem bestimmten Grenzwert liegt, zu einem qualitätsgesicherten Plasmapool vereinigt werden und diejenigen Einzelspenden, von denen die detektierte Menge an viralen Genomen oder Genomsequenzen größer oder gleich dem Grenzwert ist, einer Weiterbehandlung zugeführt oder ausgeschieden werden und das Verfahren solange wiederholt wird, bis die Anzahl der Einzelspenden, welche weiterzubehandeln oder auszuscheiden sind, eine festgelegte Zahl erreicht oder unterschritten hat.

3. Verfahren zur Herstellung eines qualitätsgesicherten Arzneimittels, enthaltend ein oder mehrere Plasmaproteine, umfassend die Schritte:
- Bereitstellen eines Plasmapools durch ein Verfahren nach Anspruch 1 oder 2 und
- Aufarbeitung dieses Plasmapools unter Einbeziehung von mindestens noch einem wesentlichen Virusabreicherungs- bzw. Virusinaktivierungsschritt zu einem qualitätsgesicherten Arzneimittel.

4. Verfahren nach einem der Ansprüche 1 oder 2, **dadurch gekennzeichnet, dass** der definierte Grenzwert der nicht zu überschreitenden Virusbelastung des Plasmapools an vermehrungsfähigen haematogenen Viren auf maximal 500 Genomäquivalente, insbesondere maximal 200 Genomäquivalente, bevorzugt 100 Genomäquivalente, besonders bevorzugt 50 Genomäquivalente, pro ml Ausgangsmaterial limitiert ist.

5. Verfahren nach einem der Ansprüche 1 bis 4, **dadurch gekennzeichnet, dass** der Plasmapool bzw. die darin enthaltenen Genomäquivalente konzentriert werden, vorzugsweise durch Lyophilisation, Adsorption oder Zentrifugation.

6. Verfahren nach einem der Ansprüche 1 bis 5, **dadurch gekennzeichnet, dass** für mehrere Viren spezifische Primer-Paare bei der Amplifikation eingesetzt werden, so dass die Anwesenheit mehrerer verschiedener Viren gleichzeitig untersucht werden kann.

7. Verfahren nach einem der Ansprüche 1 bis 6, **dadurch gekennzeichnet, dass** n gleich 2 bis 200.000, vorzugsweise 20 bis 20.000, besonders bevorzugt 200 bis 2000, insbesondere 200, m zwischen 1 und 100.000, vorzugsweise zwischen 2 und 1000, und die festgelegte Zahl an schließlich auszuscheidenden Einzelspenden gemäß Anspruch 2 die Zahl 100, vorzugsweise 10, besonders bevorzugt 1, ist.

8. Verfahren nach einem der Ansprüche 1 bis 7, **dadurch gekennzeichnet, dass** die zu einem qualitätsgesicherten Plasmapool vereinigten Einzelspenden mit weiteren qualitätsgesicherten Plasmapools vereinigt werden, so dass ein qualitätsgesicherter Minipool, Makropool oder Multimakropool zur Verfügung gestellt wird.

## Claims

1. A method of preparing a plasma pool from approximately 2,000 to 200,000 individual donations for fractionation, **characterised by** the following method steps:
(a) testing virus-neutralising antibodies against the viruses to be examined or testing a protective immunity in the donor of the starting material, and
(b) determining the genome equivalents of HIV and HCV capable of reproduction, as well as optionally further hematogenous viruses capable of reproduction, in particular HAV and HBV,
- taking samples from n individual donations,
- combining the individual donation samples to m sample pools and
- detecting the amount of viral genomes or genome sequences present in these sample pools by means of a quantifiable, controlled and non-inhibited PCR (polymerase chain reaction) method for the detection or respectively determination of nucleic acids, wherein the determined amount of genome equivalents only gives the true value of the load of viruses capable of reproduction only when all genome equivalents originate from active viruses, and wherein possibly present inhibitors of the PCR reaction are removed or depleted in the plasma pool before the PCR method;
wherein before or while carrying out the PCR method, one or several internal standards or reference preparations, respectively, are added to the sample, the standards being determined or detected, respectively, in one and the same test vessel simultaneously with viral genomes or genome sequences possibly present;
whose detected quantity of viral genomes or genome sequences in the sample pool lies below a specified limiting value are combined into a quality-assured plasma pool, and those individual donations (nₐ), whose detected amount of viral genomes or genome sequences in the sample pool is larger than or equal to the limiting value, are subjected to a further treatment or are eliminated, n and m being positive integers.

2. A method according to claim 1, **characterised in that**
- samples are taken once more for the further treatment of the eliminated nₐ individual donations and these individual donation samples are combined to mₐ sample pools, nₐ and mₐ being positive integers with mₐ ≥ 2, and the ratio of mₐ : nₐ being larger than the ratio of m : n and
- the amount of viral genomes or genome sequences in these sample pools is detected once more by means of a quantifiable, controlled and non-inhibited method for the detection or determination, respectively, of nucleic acids,
whereupon those individual donations, whose detected amount of viral genomes or genome sequences in the sample pool lies below a certain limiting value, are combined into a quality-assured plasma pool and those individual donations, whose detected amount of viral genomes or genome sequences is larger than or equal to the limiting value, are subjected to a further treatment or are eliminated, and the method is repeated until the number of individual donations, which are to be treated further or eliminated, has reached or fallen below a set number.

3. A method for the preparation of a quality-assured drug containing one or more plasma proteins, comprising the steps of
- providing a plasma pool by a method according to claim 1 or 2, and
- processing this plasma pool to a quality-assured drug by including at least one further substantial virus depletion or virus inactivation step., respectively

4. A method according to , **characterised in that** the defined limiting value of the viral load of the plasma pool by hematogenous viruses capable of reproduction which is not to be exceeded is limited to a maximum of 500 genome equivalents, particularly to a maximum of 200 genome equivalents, preferably to 100 genome equivalents, especially preferably to 50 genome equivalents per ml of starting material.

5. A method according to any one of claims 1 to 4, **characterised in that** the plasma pool or the genome equivalents contained therein, respectively, are concentrated, preferably by lyophilization, adsorption or centrifugation.

6. A method according to any one of claims 1 to 5, **characterised in that** primer pairs specific to several viruses are used in the amplification, so that the presence of several different viruses can be assayed simultaneously.

7. A method according to anyone of claims 1 to 6, **characterised in that** n is equal to 2 to 200,000, preferably to 20 to 20,000, particularly preferably to 200 to 2,000, and especially to 200, m is between 1 and 100,000, preferably between 2 and 1,000, and that the set number of individual donations finally to be excluded in accordance with claim 2 is the number 100, preferably 10, particularly preferably 1.

8. A method according to any one of claims 1 to 7, **characterised in that** the individual donations combined into a quality-assured plasma pool, are combined with further, quality-assured plasma pools, so that a quality-assured minipool, macropool or multimacropool is provided.

## Revendications

1. Procédé de fabrication d'un pool plasmatique d'environ 2000 à 200 000 dons individuels en vue d'un fractionnement, **caractérisé par** les étapes de traitement suivantes :
(a) test sur les anticorps neutralisant les virus à l'égard des virus considérés ou vérification d'une immunité protectrice chez le donneur du matériau de départ et
(b) détermination des équivalents génome des VIH et VHC capables de proliférer, ainsi que le cas échéant, d'autres virus hématogènes capables de proliférer, en particulier le VHA et le VHB,
- prélèvement d'échantillons auprès de n donneurs individuels,
- mise en commun des échantillons des donneurs individuels en m pools d'échantillons et
- détection des quantités de génomes viraux ou de séquences génomiques présents dans ces pools d'échantillons au moyen d'un procédé de PCR (réaction en chaîne polymérase) quantifiable, contrôlable et non inhibé pour prouver, voire déterminer la présence d'acides nucléiques, les quantités d'équivalents génome déterminées ne donnant la valeur réelle de la charge de virus capables de proliférer que lorsque tous les équivalents génome proviennent de virus actifs et les inhibiteurs éventuellement présents de la réaction de PCR sont éloignés du procédé de PCR ou divergents et avant ou pendant le déroulement du procédé, un ou plusieurs standards internes ou préparations de référence étant ajoutées à l'échantillon, les standards étant déterminés ou mis en évidence en même temps avec des génomes viraux éventuellement présents ou des séquences génomiques dans un seul et unique récipient de test,
après quoi les dons individuels (nₐ), dont les quantités de génomes viraux ou de séquences génomiques détectées dans le pool d'échantillon sont en dessous d'une certaine valeur seuil, sont rassemblés en un pool plasmatique de qualité garantie, et les dons individuels (nₐ) dont les quantités de génomes viraux ou de séquences génomiques détectées dans le pool d'échantillons sont supérieures ou égales à la valeur seuil, sont soumis à un autre traitement ou sont exclus et où n et m sont des nombres entiers positifs.

2. Procédé selon la revendication 1, **caractérisé en ce que**
- de nouveaux échantillons sont prélevés pour le traitement supplémentaire des dons individuels nₐ exclus et ces échantillons de dons individuels sont rassemblés en un pool d'échantillons mₐ, où nₐ et mₐ sont des nombres entiers positifs, mₐ est ≥ 2 et le rapport mₐ : nₐ est supérieur au rapport m:n, et
- des quantités de génomes viraux ou de séquences génomiques présents dans ces pools d'échantillons sont détectées au moyen d'un procédé quantifiable, contrôlable et non inhibé pour prouver, voire déterminer la présence d'acides nucléiques,
après quoi les dons individuels, dont les quantités de génomes viraux ou de séquences génomiques détectées dans le pool d'échantillons sont en dessous d'une certaine valeur seuil, sont rassemblés en un pool plasmatique de quantité garantie et les dons individuels, dont les quantités de génome viraux ou de séquences génomiques détectées dans le pool d'échantillons sont supérieures ou égales à la valeur seuil, sont soumis à un autre traitement ou sont exclus et le procédé se poursuit jusqu'à ce que le nombre de dons individuels, qui est à traiter à nouveau ou à exclure, atteigne ou soit inférieure à un nombre déterminé.

3. Procédé de fabrication d'un médicament de qualité garantie, contenant une ou plusieurs protéines plasmatiques, comprenant les étapes de :
- préparation d'un pool plasmatique grâce à un procédé selon la revendication 1 ou 2 et
- modification de ce pool plasmatique par la prise en compte d'au moins une étape essentielle d'affaiblissement ou d'inactivation du virus en vue d'un médicament de qualité garantie.

4. Procédé selon l'une quelconque des revendications 1 ou 2, **caractérisé en ce que** la valeur seuil définie de la charge virale du pool plasmatique à ne pas dépasser pour les virus hématogènes capables de proliférer est limitée à un maximum de 500 équivalents génome, en particulier un maximum de 200 équivalents génome, de préférence de 100 équivalents génome, de façon particulièrement préférée, de 50 équivalents génome, par ml de matériau de départ.

5. Procédé selon l'une des revendications 1 à 4, **caractérisé en ce que** le pool plasmatique, voire des équivalents génome contenus dans celui-ci, et concentré par lyophilisation, adsorption ou centrifugation.

6. Procédé selon l'une des revendications 1 à 5, **caractérisé en ce que** des paires d'amorces spécifiques pour plusieurs virus sont utilisées lors de l'amplification, de sorte que la présence de plusieurs virus différents peut être examinée simultanément.

7. Procédé selon l'une des revendications 1 à 6, **caractérisé en ce que** n va de 2 à 200 000, de préférence de 20 à 20 000, de façon particulièrement préférée, de 200 à 2000, en particulier 200, que m est entre 1 et 100 000, de préférence entre 2 et 1000, et que le nombre déterminé de dons individuels finalement exclus correspond selon la revendication 2, à 100, de préférence 10, de façon particulièrement préférée, 1.

8. Procédé selon l'une des revendications 1 à 7, **caractérisé en ce que** les dons individuels rassemblés en un pool plasmatique de qualité garantie sont mis en commun avec un autre pool plasmatique de qualité garantie, de sorte qu'un minipool, macropool ou multi-macropol soient proposés.
